# EUROPEAN PATENT SPECIFICATION

(11) **EP 1 720 479 B1**
(45) Date of publication and mention of the grant of the patent: **23.04.2014**
(21) Application number: 05717942.6
(22) Date of filing: 07.03.2005
(51) Int. Cl.: A61B 19/00

(54) **REGISTRATION METHODS AND APPARATUS**
REGISTRIERVERFAHREN UND -GERÄT
PROCEDE ET DISPOSITIF DE REPERAGE

(30) Priority: 05.03.2004 GB 0405011; 01.06.2004 US 575402 P
(43) Date of publication of application: 15.11.2006
(73) Proprietor: Depuy International Limited, Beeston, Leeds LS11 8DT (GB)
(72) Inventor: REVIE, Ian, Boroughbridge YO51 9AX (GB); ASHBY, Alan, York YO30 6LN (GB); SLOMCZYKOWSKI, Mike, Harrogate HG2 9QG (GB)
(74) Representative: Alton, Andrew
(86) International application number: PCT/GB2005/000874
(87) International publication number: WO 2005/086062

(56) References cited:
- FR-A- 2 798 760
- US-A1- 2002 087 062
- US-A1- 2002 188 194
- US-A1- 2003 088 179
- US-A1- 2003 179 856
- US-B1- 6 246 900
- US-B1- 6 640 127

## Description

The present invention relates to methods and apparatus for use in registering images of body parts with body parts, and in particular to methods, systems, apparatus, computer program code and computer program products for producing registered images for use in image guided surgical procedures, and in particular orthopaedic procedures.

Various methods can be used to register images of body parts with the reference frames of tracking systems. However, many of these require the presence of imaging systems within the operating theatre which facilities are not necessarily available.

US 6,640,127 describes a reference frame in the form of spectacles having a plurality of markers which are worn by a patient. The markers are made of a material that can be clearly imaged by X-ray or MRI scan. A surgical operation navigation unit is used together with an imaging unit. Co-ordinate systems are defined for surgical instruments (E), for captured images (P) and for real space (W) in the operating room. The different co-ordinate systems can be registered using various different types of transformation matrices. The image co-ordinate system (P) is registered with the real space co-ordinate system of the patient (W) in a process referred to as Operative Calibration. Co-ordinate transformation parameters _{W}H_{µ} define the co-ordinate transformation from the image co-ordinate system to the real world co-ordinate system. The reference frame is worn by the patient and the patient is imaged so as to capture the markers in the image. Then the positions of the markers in the imaging co-ordinate system are determined. Then the positions of the markers are detected in the real world co-ordinate system using a position sensing system. Then the image can be aligned with the patient in the real world, by registering the image co-ordinate system and the real world co-ordinate system using the marker positions captured in the image and the marker positions determined by the position sensing system. Hence, this approach determines the transformation required between an imaging system co-ordinate system and a different, real world co-ordinate system in order to register the patient image in the real world. Further the registration is based solely on the markers present in the image captured by the imaging system.

FR 2798760 describes a method and system for instantiating statistical bone models using captured bone images. A patient can have a sensor attached to their bone and sensors are also attached to the X-ray camera and detector of a C-arm. A localiser is used to capture the position of the patient's bone, the X-ray camera and the detector. The position of the marker attached to the patient defines a reference referential-system with respect to which the positions of other parts in the referential-system of the localiser are determined. A further sensor can be attached to a patient support in cases where the localiser cannot easily detect the X-ray camera and the detector. In that case, the position of the X-ray camera and detector are defined relative to the position of the further sensor attached to the patient support in the referential-system of the localiser. A computer system controls the localiser and X-ray image capture. At least one two-dimensional view of the patients bone is captured and the contours of the bone image are used to search a database to match to a three-dimensional model of the bone, which may be a deformable statistical model. Back projected X-rays being tangential to the two-dimensional image contours is used as the criterion for matching.

US 2002/0188194 describes a system and method for positioning and repositioning a part of a patient's body with respect to radiation therapy machine or MRI scanner using multiple cameras to view the body and the radiation therapy machine or MRI scanner. Index markers are identified and located by the cameras in 3D space. The index markers are in a determinable relationship to analogous markers used during previous image scanning of the patient. Anatomical targets determined from image scanning can be located relative to reference positions associated with the treatment or diagnostic machine. X-ray imaging of the patient can further refine anatomical target positioning relative to the treatment or diagnostic imaging reference point.

The present invention therefore relates to being able to provide an image in a format in which the image can be registered or is already automatically registered for use in a surgical procedure.

The invention is defined in appended independent claims 1, 22 and 37. Preferred embodiments are described in the dependent claims.

According to a first aspect of the present invention, there is provided a method for generating and displaying a registered image of a body part of a patient for use in a computer aided surgical procedure, the method comprising: capturing at least a first image of the body part using an imaging system at a first location, wherein a first marker detectable by a tracking system having a reference frame is attached to part of the imaging system; detecting the position of a second marker, which has been attached to the body part prior to any surgical steps of the surgical procedure, while the at least a first image is being captured using the tracking system; detecting the position of the first marker while the at least a first image is being captured using the tracking system; obtaining an indication of the position of the first image in the reference frame of the tracking system using the detected position of the first marker and a known positional relationship between the first marker and an image plane of the imaging system; determining a mapping to bring the first image into registration with the position of the body part in the reference frame using the detected position of the second marker in the reference frame; detecting the position of the second marker while the body part is in an operating theatre, being a different location to the first location, using either the tracking system which has been moved to the operating theatre from the first location, or a second tracking system and determining the position of the body part in the reference frame of the tracking system or second tracking system; and displaying the registered image by using the mapping to register the first image with the detected position of the body part in the operating theatre while the body part is in the operating theatre.

In this way, the invention provides an image in a form registrable with the position of the body part and so a registered image can be generated for use in a surgical procedure before any invasive acts associated with the surgical procedure are carried out. Hence the image is pre-registered or automatically registered, or sufficient data exists that the image is in a condition that the image can be brought into registration with the position of a body part when required for use in the computer aided surgical procedure.

The surgical procedure can be an orthopaedic procedure. The orthopaedic procedure can be carried out on a knee or hip and can include implanting an orthopaedic prosthetic implant or implants.

The marker can be wirelessly detectable by the tracking system, and the marker can be wirelessly detectable at radio frequencies. The marker can be wire based. The marker can be acoustically detectable, e.g. by ultrasound, or electromagnetically detectable, including using infra-red radiation.

The imaging system can be an X-ray system. The X-ray system can be a fluoroscopic X-ray system. The X-ray system can include an x-ray detector. The x-ray detector can have an imaging plane. The x-ray detector can be an x-ray cassette. The x-ray detector can be a digital or an electronic detector which can generate a digital image or digital image signal.

The position of the marker can be detected with the patient standing. The position of the marker can be detected with the patient's weight imparting a load on the body part being imaged.

Obtaining an indication of the position of the at least first image relative to the reference frame of the tracking system can include determining the position of an X-ray detector in the reference frame of the tracking system.

The method can further comprise capturing a second image of the body part using the X-ray system. The second image can be in a second direction different to a first direction in which the first image was captured. A three dimensional image of the body part can be derived from the first and second images. A three dimensional image of the body part in an orientation of the body part corresponding to the orientation of the patient during the surgical procedure can be generated from the first and second images.

Capturing the second image can include moving the patient relative to the X-ray system. Capturing a second image can include moving an X-ray source relative to the patient. The method can further comprises determining the position of the X-ray source in the reference frame of the tracking system when the first and/or second image is captured. Determining the position of the X-ray source can include wirelessly tracking the position of a marker attached to the x-ray source at radio frequencies. The first image can be captured using a first X-ray source. Capturing the second image can include using a second X-ray source at a second position which is different to a first position of the first X-ray source. The or each x-ray source can be an x-ray camera.

The method can further comprising generating a three dimensional image of the body part from the first and second images.

The method can further comprise determining the distance between the body part and an imaging plane of an X-ray detector along a direction perpendicular to the plane of the imaging plan. The distance can be used to compensate the first image captured by the X-ray detector for linear magnification.

The imaging system can be a CT scan or an MR scan system.

The body part of the patient can be located on a patient support part of the imaging system when the first image is captured. The patient support part can be a table. The method can further comprise determining the position of the patient support part or a part of the patient support part in the reference frame of the tracking system. The method can further include determining the position of the patient support part relative to an imaging plane or region of the imaging system.

The method can further comprising mounting a marker detectable by the tracking system on the patient support part.

The body part of the patient can be located on a patient support part of the imaging system when the first image is captured. The method can further comprise determining the position of an imaging plane or imaging region of the scan system relative to the position of the patient support part or a part of the patient support part.

The first image can include the marker or a part of the marker and at least a part of the body part. The position of the marker can be detected when the first image is captured.

Each marker can identify itself uniquely. Each marker can indicate its position and/or its orientation. Only one marker per bone can be needed in order to determine the position and/or orientation of the body part in the reference frame of the tracking system.

Determining the mapping or mapping the first image into registration with the position of the body part can include using the position of the further marker or markers. Determining the mapping can include identifying a vector in the reference frame of the tracking system. The mapping can be the opposite vector to the identified vector.

According to a second aspect of the invention there is provided a system for generating and displaying a registered image of a body part of a patient for use in an image guided surgical procedure, the system including: a tracking system for detecting markers and determining the position of the markers in a reference frame of the tracking system; an imaging system at a first location for capturing at least a first image of the body part, wherein a first marker detectable by the tracking system is attached to a part of the imaging system; a second marker attachable to the body part of the patient and detectable by the tracking system; and a computer control system configured to: cause the imaging system to capture at least a first image of the body part; cause the tracking system to detect the position of the second marker when attached to the body part while the at least first image is being captured at the first location; cause the tracking system to detect the position of the first marker while the at least first image is being captured; obtain an indication of the position of the first image in the reference frame of the tracking system using the detected position of the first marker and a known positional relationship between the first marker and an image plane of the imaging system; determine how to map the first image into registration with the position of the body part in the reference frame using the detected position of the second marker in the reference frame; cause either the tracking system when moved to an operating theatre, or a further tracking system in the operating theatre, to detect the position of the second marker while the body part is in the operating theatre, being a different location to the first location, and determine the position of the body part in the reference frame of the tracking system or further tracking system; and display (300, 398) the registered image by using the mapping to register the first image with the detected position of the body part in the operating theatre.

Counterpart features to the preferred features and details of the first embodiment of the invention can also be preferred features of the second aspect of the invention, and *vice versa.*

The marker can be wirelessly detectable by the tracking system. The marker can be wirelessly detectable by the tracking system at radio frequencies. The marker can be percutaneously implantable in a bone of the patient.

The marker can be mounted on an image capturing or detecting part of the imaging system.

The imaging system can be an X-ray imaging system, including an X-ray fluoroscopy imaging system. The X-ray imaging system including an X-ray source and an X-ray detector having an imaging plane. The system can further comprise a second marker detectable by the tracking system mounted on the X-ray detector. The system can further comprise a third marker detectable by the tracking system mounted on the X-ray source. The X-ray source can be movable relative to the X-ray detector. Preferably the x-ray source can be rotated or pivoted about by at least 90°.

The system can further comprise a fourth marker detectable by the tracking system and a further X-ray source. The fourth marker can be mounted on the further X-ray source.

The computer control system can be further configured to determine a separation between the marker and an imaging plane of the X-ray detector and to use the separation to correct the first image.

The imaging system can be a CT scan or MR scan imaging system.

The imaging system can includes a patient support part. The further marker can be mounted on the patient support part.

The computer control system can be further configured to determine the position of the patient when the first image includes the marker or at least a part of the marker and at least a part of the body part.

The imaging system can include a patient support part. The computer control system can determine the position of an imaging plane or region of the imaging system relative to the patient support part.

The system can further comprise a further marker or markers attachable to a further body part or body parts of the patient and detectable by the tracking system.

According to a third aspect of the invention, there is provided a computer implemented method for generating and displaying a registered image of a body part of a patient bearing a second marker detectable by a tracking system having a reference frame, the registered image being for use in an image guided surgical procedure and being generated prior to any surgical steps of the image guided surgical procedure, the method comprising: determining the position of the second marker in the reference frame, from the position of the second marker tracked by the tracking system while a first image of the body part was being captured by an imaging system at a first location; determining the position of a first image of the body part in the reference frame of the tracking system, from the position of a first marker attached to a part of the imaging system and tracked by the tracking system while the first image of the body part was being captured and a known positional relationship between the first marker and an image plane of the imaging system; determining a mapping to bring the first image into registration with the position of the body part in the reference frame using the detected position of the second marker in the reference frame; determining the position of the body part in the reference frame of the tracking system or a second tracking system from the position of the second marker while the body part is in an operating theatre, being a location different to the first location, and tracked by either the tracking system which has been moved to the operating theatre or a second tracking system in the operating theatre; and displaying the registered image by using the mapping to register the first image with the detected position of the body part in the operating theatre.

Counterpart features to the preferred features and details of the first and second embodiment of the invention can also be preferred features of the third aspect of the invention, and *vice versa.*

The mapping can be a vector in the reference frame of the tracking system.

The registered image can be derived from the captured image. The registered image can be a rendered image showing the body part at a position and/or orientation different to that of the body part when the image was captured.

The first image can include a further body part of the patient bearing a further marker detectable by the tracking system. The method can further comprise determining the position of the further marker in the reference frame.

Determining the position of the first image can include determining the position of an imaging plane or region of the imaging system relative to a patient support part of the imaging system.

Determining the position of a first image of the body part relative to the reference frame can include determining the position of an image of at least a part of the marker in the first image.

The registered image can be displayed during a computer aided surgical procedure. The registered image can be displayed during a computer aided surgical procedure and before any invasive steps associated with the surgical procedure have been carried out.

According to a fourth aspect of the invention, there is provided a computer readable medium bearing computer program code executable by a data processing device to provide a method according to the third aspect of the invention.

An embodiment of the invention will now be described, by way of example only, and with reference to the accompanying drawings, in which:
Figure 1 shows a flow chart illustrating at a high level a patient treatment method in which methods of the invention can be used;
Figure 2 shows a schematic block diagram of a tracking system and a marker part of a system of the invention;
Figure 3 shows a perspective view of the marker shown in Figure 2;
Figure 4 shows a housing part of an implantable marker part of the system of the invention and useable in methods of the invention;
Figure 5 shows a schematic diagram of an embodiment of the system according to the invention including the tracking system shown in Figure 2 and an X-ray imaging system;
Figure 6 shows a flow chart illustrating a method of using the system shown in Figure 5 according to the invention;
Figure 7 shows a schematic illustrating of the display of a registered image in an operating theatre as part of an image guided surgical procedure;
Figure 8 shows a flow chart illustrating a method of providing the registered image as part of an image guided surgical procedure;
Figure 9 shows a schematic diagram of a further embodiment of the system according to the invention including the tracking system shown in Figure 2 and a CT imaging system;
Figure 10 shows a flow chart illustrating a method of using the system shown in Figure 9 according to the invention;
Figure 11 shows a flow chart illustrating a further method of using the system shown in Figure 9 according to the invention;
Figure 12 shows a flow chart illustrating a further method of providing the registered image as part of an image guided surgical procedure;
Figure 13 shows a schematic software architecture of a computer control part of the system of the invention;
Figure 14 shows a flow chart illustrating a computer implemented method of providing a registered body part image according to the invention;
Figure 15 shows a graphical representation of the registration method illustrated in Figure 14; and
Figure 16 shows a schematic block diagram of a computer part of the system of the invention.

Similar items in different Figures share common reference numerals unless indicated otherwise.

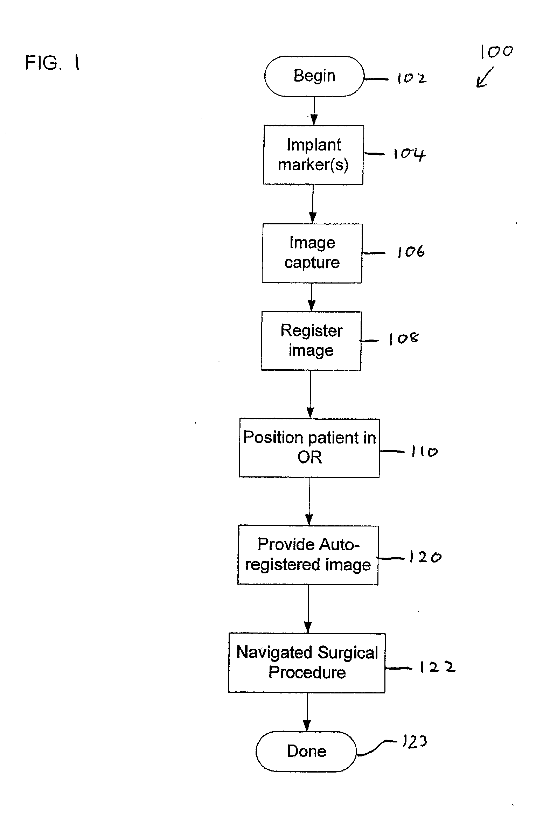

With reference to Figure 1 there is shown a flowchart illustrating, at a high level, a general method 100 in which the registration method of the present invention can be utilised. The present invention allows a registered image to be provided for use in a computer aided surgical procedure, in which images of the patient's body parts are pre-registered, or automatically registered and available for use in the surgical procedure. The method is particularly advantageous as the patient's body parts images are registered without requiring any invasive surgical procedure to be carried out on the patient. This therefore reduces the time of, and complexity of, the computer aided surgical procedure. The image registration method can be carried out as an out patient or clinical procedure some time before the computer aided surgical procedure, e.g. several days or weeks, or immediately prior to the surgical procedure.

The method begins at step 102 and at step 104 markers detectable by a checking system so as to determine the position of the markers are attached to the patient's body part. Various types of markers and associated tracking technology can be used. For example, an acoustic or ultrasound based tracking system can be used. Or alternatively, a wire based tracking system can be used. In other embodiments, various wireless tracking technologies can be used, such as an infrared based tracking technology, which uses passive markers having infrared reflective spheres. A suitable infrared based tracking technology is available from BrainLab GmbH of Germany.

In particularly preferred embodiment of the invention, a wireless radio frequency based tracking technology is used and the markers are implanted within the bones of the patient through the patient's skin. Suitable percutaneously implantable markers and an associated tracking system are described in greater detail below.

At step 104, a marker is implanted in each of the bones for which a registered image is to be provided during the subsequent surgical procedure. The invention will be described below with reference to a total knee replacement procedure involving prosthetic orthopaedic implants, but it will be appreciated that the method is not limited to that surgical procedure nor to implanting orthopaedic prostheses. Rather, the method of the present invention is useable in any computer aided procedure in which registration of image data relating to a body part with the reference frame of a tracking system is desirable.

At step 106, after the markers have been implanted, an image or several images of the body part, e.g. the femur, knee, tibia and fibia are captured using an imaging system. After the body part images have been captured, at step 108, the body part images, or images of the body part derived from the captured images, are brought into registration with the actual position of the body part, e.g. mapping images of the femur, knee, tibia and fibia into registration with the detected position of the femur, the tibia and fibia. It is not necessary to actually generate a registered image at step 108, but merely to carry out the data processing operations required in order to allow a registered image to be generated, either for display, or to be used or otherwise processed by a computer aided surgical system during the surgical procedure.

After the body part position data and image data have been processed to allow a registered image to be provided at step 108, at step 110, the patient can be located at 110 in the operating room in which the surgical procedure is to be carried out. It will be appreciated that pre-operative steps 104, 106, 108 can be carried out some time e.g. days, weeks, months before the subsequent steps of the operation are carried out. Alternatively, the pre-operative steps can be carried out a matter of hours or minutes before the operation itself is carried out. It will also be appreciated that in a suitably equipped operating room, it will be possible to carry out steps 104 t 108 in the operating room in which case step 110 corresponds to making the patient available for surgery and merely indicates the cross over between he pre-operative steps and the beginning of the steps associated with the surgical procedure. Therefore step 110 does not necessarily require the actual physical movement of the patient in some embodiments of the invention. However, it will be appreciated that no invasive surgical steps have been carried out and that invasive surgical steps only begin later on in the method at step 122.

After the patient has been made available for surgery in the operating room at step 110, at step 120, the registered image can be made available to a computer aided surgical system in the operating room and either displayed by the system, used or otherwise processed by the computer aided surgical system so as to be of assistance in the subsequent computer aided surgical procedure. Then at step 122, the computer aided surgical procedure is begun and can include various steps, including planning the surgical procedure or the navigated use of tools, instruments and implants and the provision of images of the body part in image guided steps of the surgical procedure. The above will e referred to herein generally as computer aided, navigated or image guided surgical steps or methods unless the context implies a more specific meaning. However, all of these share the feature of using an image or image data associated with a body part which has been registered with the actual position of the body part so as to assist in the carrying out of a surgical or other treatment step or operation.

The registered image of the body part can be used in step 122 to aid in the planning of the knee replacement surgery, e.g. by assessing the current kinematic performance of the patient's knee and identifying appropriate positions for the femoral and tibial orthopaedic implants. The navigated guidance of instruments to prepare the patient's knee for the implants which can also involve displaying captured images of the patient's bones together with representations of the tools, and the navigated and image guided positioning of the implants in the patient's knee and finally an assessment of the kinematic performance of the patient's knee after the orthopaedic implants have been implanted. The method 100 then ends at step 123 after the surgical procedure has been completed and when the immediate post-operative assessment of the success of the procedure has been carried out.

It will be appreciated that the method of the present invention is not limited to surgical procedures involving orthopaedic prosthetic implants and it can be of benefit in other surgical procedures in which registered images of the patient's bones would be useful, such as other orthopaedic procedures, including, by way of example only, a cruciate knee ligament replacement procedure. The method is also particularly suitable for use in hip, elbow, shoulder and spinal procedures, and especially those including the implantation of prosthetic and other implants in those joints or bone structures.

A suitable marker 140 and associated tracking system 130 for use in the image registration method will briefly be described in greater detail. Aspects of the marker 140 and tracking sub-system 130 are described in greater detail in U.S. patent publication no. US 2003/0120150 A1 (U.S. patent application serial no. 10/029,473).

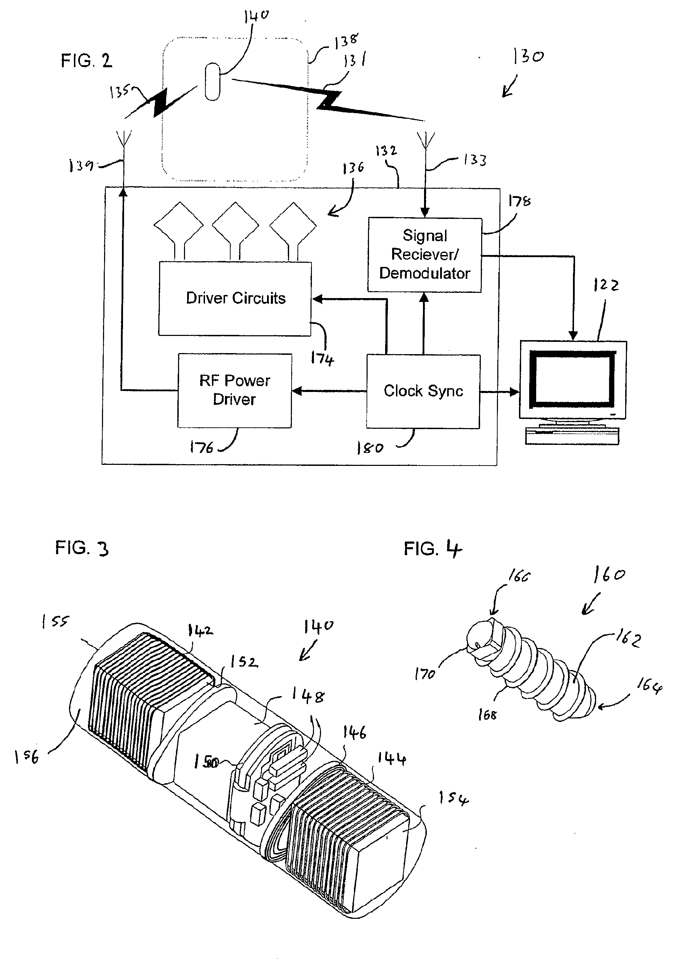

With reference to Figures 2, 3 and 4 there are shown a schematic block diagram of the magnetic tracking system 130, marker, or wireless position sensor, 140, which can be tracked by the tracking system, and a housing 160 for the marker which provides a percutaneously implantable marker. The marker 140 generates and wirelessly transmits a digital signal 131 encoding data items indicative of the marker's identity, its location (x, y and z co-ordinates within the cartesian reference frame of the tracking system) and orientation (pitch, roll and yaw), in response to an external magnetic field 138 produced by the three field generator coils 136 (also referred to as radiator coils). The location and orientation of the marker will generally be referred to as the markers position.

Figure 2 illustrates some of the circuitry in a tracking station part13 of the tracking system 130 which co-operates with computer based controller 122. Field generator coils 136 are driven by driver circuits 174 to generate electromagnetic fields at different, respective sets of frequencies {w₁}, {w₂} and {w₃}. Typically, the sets comprise frequencies in the approximate range of 100 Hz - 0 kHz, although higher and lower frequencies may also be used. In one embodiment frequencies in the range of between approximately 15 kHz - 0 kHz are used. The sets of frequencies at which the coils radiate are set by computer 122, which serves as the system controller for the tracking system 130. The respective sets of frequencies may all include the same frequencies, or they may include different frequencies. In any case, computer 122 controls circuits 174 according to a known multiplexing pattern, which provides that at any point in time, no more than one field generator coil is radiating at any given frequency. Typically, each driver circuit is controlled to scan cyclically over time through the frequencies in its respective set. Alternatively, each driver circuit may drive a respective one of coils 136 to radiate at multiple frequencies simultaneously.

For the purposes of tracking station 132, coils 136 may be arranged in any convenient position and orientation, so long as they are fixed in respect to some reference frame, and so long as they are non-overlapping, that is, there are no two field generator coils with the exact, identical location and orientation. Typically, for surgical or during patient image capturing applications the coils are located in a triangular arrangement. The coil axes may be parallel, or they may alternatively be inclined. Bar-shaped transmitters or even triangular or square-shaped coils could also be useful for such applications.

It is desirable that coils 13 6 be positioned away from the surgical or image capturing field, so as not to interfere with the surgeon's freedom of movement or with the patient or a patient support. On the other hand, the coils should be positioned so that the working volume 138 of the tracking system includes the entire area in which the surgeon is operating or in which the patient's marked body parts are on in which any marked parts of the imaging system are located. At the same time, the locations and orientations of coils 136 should be known relative to a given reference frame in order to permit the coordinates of markers 140 to be determined in that reference frame. In practice, coils 136 are mounted on a reference structure part (not shown) of the tracking station 134.

The markers 140 include sensor coils 142, in which electrical currents are induced to flow in response to the magnetic fields produced by field generator coils 136. The sensor coils 142 may be wound on either air cores or cores of magnetic material. Typically, each marker comprises three sensor coils, having mutually orthogonal axes, one of which is conveniently aligned with a principal axis of the housing 160 or of an orthopaedic implant, such as a longitudinal axis. The three coils may be concentrically wound on a single core, or alternatively, the coils may be non-concentrically wound on separate cores, and spaced along the principal axis. The use of non-concentric coils is described, for example, in the PCT Patent Publication WO 96/05768 and in the corresponding U.S. Patent Application 09/414,875.

Alternatively, the markers 140 may each comprise only a single sensor coil or two sensor coils. Further alternatively, markers 140 may comprise magnetic position sensors based on sensing elements of other types known in the art, such as Hall effect sensors.

At any instant in time, the currents induced in the sensor coils 142 comprise components at the specific frequencies in sets {w₁}, {w₂} and {w₃} generated by field generator coils 136. The respective amplitudes of these currents (or alternatively, of time-varying voltages that maybe measured across the sensor coils) are dependent on the location and orientation of the marker relative to the locations and orientations of the field generator coils. In response to the induced currents or voltages, signal processing and transmitter circuitry in each marker generate and transmit signals 131 that are indicative of the location and orientation of the sensor. These signals are received by receiving antenna 133, which is coupled to computer 122 via signal receiver and demodulation circuitry 178. The computer processes the received signals, together with a representation of the signals used to drive field generator coils 136, in order to calculate location and orientation coordinates of the implantable marker. The coordinates are processed and stored by the computer 122 as will be described in greater detail below.

Although in Figure 2 tracking system 130 is shown as comprising three field generator coils 136, in other embodiments, different numbers, types and configurations of field generators and sensors may used. A fixed frame of reference may be established, for example, using only two non-overlapping field generator coils to generate distinguishable magnetic fields. Two non-parallel sensor coils may be used to measure the magnetic field flux due to the field generator coils, in order to determine six location and orientation coordinates (X, Y, Z directions and pitch, yaw and roll orientations) of the sensor. Using three field generator coils and three sensor coils, however, tends to improve the accuracy and reliability of the position measurement.

Alternatively, if only a single sensor coil is used, computer 122 can still determine five position and orientation coordinates (X, Y, Z directions and pitch and yaw orientations). Specific features and functions of a single coil system (also referred to as a single axis system) are described in U.S. Patent 6,484,118.

When a metal or other magnetically-responsive article is brought into the vicinity of an object being tracked, the magnetic fields in this vicinity are distorted. There can be a substantial amount of conductive and permeable material in a surgical or imaging environment, including basic and ancillary equipment (operating tables, carts, movable lamps, etc.), invasive surgery apparatus (scalpels, scissors, etc.), and parts of the imaging system, such as X-ray sources and detectors and parts of a CT scanner and patient table. The magnetic fields produced by field generator coils 136 may generate eddy currents in such articles, and the eddy currents then cause a parasitic magnetic field to be radiated. Such parasitic fields and other types of distortion can lead to errors in determining the position of the object being tracked.

In order to alleviate this problem, the elements of the tracking station 132 and other articles used in the vicinity of the tracking system 130 are typically made of non-metallic materials when possible, or of metallic materials with low permeability and conductivity. In addition, computer 122 may be programmed to detect and compensate for the effects of metal objects in the vicinity of the monitoring station. Exemplary methods for such detection and compensation are described in U.S. Patents 6,147,480 and 6,373,240, as well as in U.S. Patent Applications 10/448,289 filed May 29, 2003 (US 2004239314) and 10/632,217filed July 31, 2003, (US 2005024043).

Marker 140 in this embodiment comprises three sets of coils: sensor coils 142, power coils 144, and a communication coil 146. Alternatively, the functions of the power and communication coils may be combined, as described in U.S. Patent Application 10/029,473 (US 2003/0120150). Coils 142, 144 and 146 are coupled to electronic processing circuitry 148, which is mounted on a suitable substrate 150, such as a flexible printed circuit board (PCB). Details of the construction and operation of circuitry 148 are described in U.S. Patent Application 10/029,473 (US 2003/0120150) and in U.S. Patent Application 10/706,298 (US 2005099290).

Although for simplicity, Fig. 3 shows only a single sensor coil 142 and a single power coil 144, in practice sensor 140 typically comprises multiple coils of each type, such as three sensor coils and three power coils. The sensor coils are wound together, in mutually-orthogonal directions, on a sensor core 152, while the power coils are wound together, in mutually-orthogonal directions, on a power core 154. Alternatively, the sensor and power coils may be overlapped on the same core, as described for example in U.S. Patent Application 10/754,751 filed January 9, 2004, (US 2005151696). It is generally desirable to separate the coils one from another by means of a dielectric layer (or by interleaving the power and sensor coils when a common core is used for both) in order to reduce parasitic capacitance between the coils.

In operation, power coils 144 serve as a power source for sensor 140. The power coils receive energy by inductive coupling from external driving antenna 139 attached to RF power driving circuitry 174. Typically, the driving antenna radiates an intense electromagnetic field at a relatively high radio frequency (RF), such as in the range of 13.5 MHz. The driving field causes currents to flow in coils 144, which are rectified in order to power circuitry 148. Meanwhile, field generator coils 136 induce time-varying signal voltages to develop across sensor coils 142, as described above. Circuitry 148 senses the signal voltages, and generates output signals in response thereto. The output signals may be either analog or digital in form. Circuitry 148 drives communication coil 146 to transmit the output signals to receiving antenna 133 outside the patient's body. Typically, the output signals are transmitted at still higher radio frequencies, such as frequencies in the rage of 43 MHz or 915 MHz, using a frequency-modulation scheme, for example. Additionally or alternatively, coil 146 may be used to receive control signals, such as a clock signal, from a transmitting antenna (not shown) outside the patient's body.

As explained above, an RF power driver 176 is provided, which drives antenna 139 to emit power signal 135, preferably in the 2-10 MHz range. The power signal causes a current to flow in power coil 144, which is rectified by circuitry 148 and used to power the markers internal circuits. Meanwhile, the electromagnetic fields produced by field generator coils 136 cause currents to flow in sensor coil 142. This current has frequency components at the same frequencies as the driving currents flowing through the generator coils 136. The current components are proportional to the strengths of the components of the respective magnetic fields produced by the generator coils in a direction parallel to the sensor coil axes. Thus, the amplitudes of the currents indicate the position and orientation of coils 142 relative to fixed generator coils 136.

Circuitry 148 measures the currents flowing in sensor coils 142 at the different field frequencies. It encodes this measurement in a high-frequency signal, which it then transmits back via antenna 146 to antenna 133. Circuitry 148 comprises a sampling circuit and analog/digital (A/D) converter, which digitizes the amplitude of the current flowing in sensor coils 142. In this case, circuitry 148 generates a digitally-modulated signal, and RF-modulates the signal for transmission by antenna 146. Any suitable method of digital encoding and modulation may be used for this purpose. Circuitry 148 also stores a unique identifier for each marker and similarly generates a digitally-modulated signal, and RF-modulates the signal 131 for transmission by antenna 146. Other methods of signal processing and modulation will be apparent to those skilled in the art.

The digitally-modulated signal transmitted by antenna 146 is picked up by receiver 178, coupled to antenna 133. The receiver demodulates the signal to generate a suitable input to signal processing circuits which can be separate to, or integrated in, the computer system 122. Typically, receiver 178 amplifies, filters and digitizes the signals from marker 140. The digitized signals are received and used by the computer 122 to compute the location and orientation of marker 140. General-purpose computer 122 is programmed and equipped with appropriate input circuitry for processing the signals from receiver 178.

Preferably, a clock synchronization circuit 180, is provided which is used to synchronize driver circuits 174 and RF power driver 176. The RF power driver can operate at a frequency that is an integer multiple of the driving frequencies of field generators 136. Circuitry 148 can then use the RF signal received by power coil 144 not only as its power source, but also as a frequency reference. Using this reference, circuitry 148 is able to apply phase-sensitive processing to the current signals generated by sensor coils 142, to detect the sensor coil currents in phase with the driving fields generated by coils 136. Receiver 178 can apply phase-sensitive processing methods, as are known in the art, in a similar manner, using the input from clock synchronization circuit 180. Such phase-sensitive detection methods enable marker 140 to achieve an enhanced signal/noise (S/N) ratio, despite the low amplitude of the current signals in sensor coils 142.

Although certain frequency ranges are cited above by way of example, those skilled in the art will appreciate that other frequency ranges may be used for the same purposes.

Circuitry 148 also stores a unique identifier for marker 140 and the unique identifier is also transmitted to the tracking system 130, so that the tracking system can determine the identity of the marker from which positional data is being received. Hence the tracking system can discriminate between different markers when multiple markers are present in the working volume 138 of the tracking station.

An advantage of using wireless markers, such as marker 140, without an on-board power source, is that the markers can be inserted in and then left inside the patient's body for later reference.

As illustrated in Figure 3, marker 140 can be hermetically sealed by encapsulation 155 in a sealant or encapsulant 156. Preferably the sealant provides any, some or all of the following shielding properties: mechanical shock isolation; electromagnetic isolation; biocompatiblility shielding. The sealant can also help to bond the electronic components of the marker together. Suitable sealants, or encapsulants, include USP Class 6 epoxies, such as that sold under the trade name Parylene. Other suitable sealants include epoxy resins, silicon rubbers and polyurethane glues. The marker can be encapsulated by dipping the marker in the sealant in a liquid state and then leaving the sealant to set or cure.

Marker 140 can be attached to orthopaedic prosthetic implants so as to allow the position of the orthopaedic implants to be tracked during a navigated or image guided surgical procedure. A housing can be provided around the encapsulant 156 and then the housed marker can be secured to the orthopaedic implant.

Figure 4 shows a housing 160 for marker 140 which can be used to provide an implantable marker which can be implanted directly in the bone of the patient. In a preferred embodiment, the implantable marker is configured to be "injectable" through the skin of the patient without requiring an ancillary incision or other preliminary surgical procedure. Housing 160 has a generally cylindrical body 162 having a tapered distal end 164 and a screw thread 168 extending along the longitudinal axis of the housing. A connector 170 in the form of a generally square shaped formation is provided at the proximal end 166 for releasably engaging with an insertion instrument so as to impart rotational drive to the housing so that the implantable marker can be screwed into the patient's bone. In one embodiment, the housing has a sharp, skin piercing tip at the distal end with a self-tapping screw thread thereon. The implantable marker can then be percutaneously implanted into the bone by pushing the marker through the skin, optionally using a guide instrument having a guide tube with a guide channel passing there along, and then screwing the implantable marker into the bone.

In another embodiment, no screw thread 168 is provided and the outer surface of the housing is substantially smooth and the distal end has a sharp bone penetrating tip, such as a trochanter tip. In this embodiment, the implantable marker can be percutaneously implanted by driving the implantable marker through the skin and pushing the implantable marker into the bone. While the screw thread provides a bone anchor for some embodiments, in this embodiment, barbs, ribs or other surface features can provide a bone anchor. Alternatively, a bone anchor can be provided by treating the outer surface of the marker housing to encourage, facilitate or otherwise promote bone on growth. For example the outer surface of the housing can be roughened or chemically treated.

In a further embodiment, the housing 160 includes a non-bone penetrating tip or nose, made of a resorbable material. Prior to implanting the marker, a hole is drilled in the bone using a guide tube and then the implantable marker is guided towards the pre-drilled hole by the guide tube and the tip self locates the implantable marker in the pre-drilled hole and then the implantable marker is screwed into the pre-drilled hole so as to drive the implantable marker into the bone.

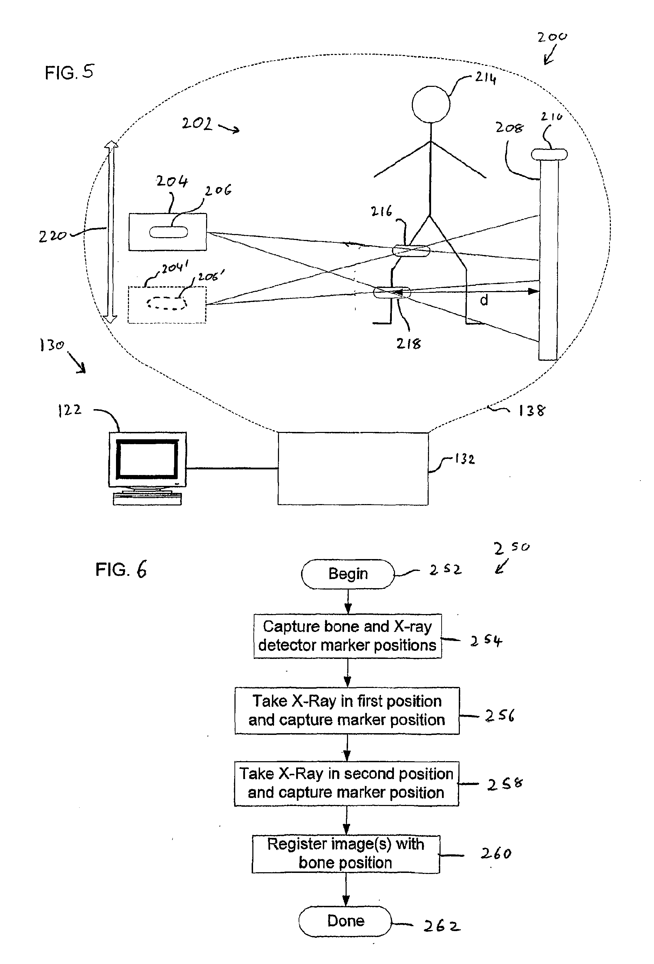

With reference to Figure 5 there is shown a system 200 according to an embodiment of the invention, including wireless tracking subsystem 130, computer control system 122 and an image system 202 in the form of an X-ray based imaging system. Imaging system 202 includes a first X-ray source 204 bearing a marker 206 trackable by the tracking system 130. Imaging system 202 also includes an X-ray detector 208 which can be in the form of an X-ray cassette including an X-ray sensitive film located at an imaging plane of the X-ray cassette. X-ray cassette 208 also bears a marker 210 detectable and trackable by the tracking system 130. The X-ray image marker 210 has a known positional relationship with the position and orientation of the imaging plane of the X-ray detector 208.

In the embodiment in which an X-ray cassette is used, the developed X-ray film can be scanned and digitised to provide the X-ray image data in a digital form. In an alternate embodiment, the X-ray detector 208 is a digital X-ray detector which generates X-ray image data directly and again the position of the X-ray image marker 310 relative to the position and orientation of the imaging plane of the digital X-ray image detector is known. There is a known positional relationship between the imaging plane of the X-ray detector 208 and the X-ray source 204.

A patient 214 having a first marker 217 detectable by the tracking system and implanted in the femur is positioned adjacent the X-ray detector. The patient 214 also has a second marker 218 implanted detectable by the tracking system implanted in their tibia. In other embodiments, only a single bone marker is implanted in a one of the patient's bones. In other embodiments, more than one marker can be implanted in each bone and more than two bones can have a one or more markers implanted therein. As indicated above, other tracking technologies can be used and the invention is not limited to the use of markers wirelessly detectable radio frequencies. For example, bone markers 216, 218 can be attached to the bone using a support structure or can be attached to the patient's body above the bone rather than within the bone.

Each of the bone markers, 218, 216 is displaced by a distance d, in a direction substantially perpendicular to the plane of the imaging plane of the X-ray detector 208. The distance d is preferably relatively small, *e.g.* a few cms and preferably in the range between approximately 10-30cm. Distance "d" is required for each image to calculate linear magnification of the X-ray image (which will later be displayed on the computer screen for navigation) with respect to the actual size of the patients bone. The same magnification factor is also applied to the navigated instruments and/or implants and their tracked movements during navigated surgery.

A method allowing images of a bone to be registered with the position of the bone will now be described with further reference to Figure 6. Figure 6 shows a flowchart illustrating a computer implemented method 250 implemented by a computer program executed by computer control system 122. At step 252, the program code is called and initiates. At step 254, the position of the bone implanted markers 216, 218 and of the X-ray image marker 210 in the reference frame of the tracking system are captured and stored. The computer program also determines the separation d between the imaging plane of the X-ray detector 208 and each of the bone implanted markers 218 and 216. Using d a linear magnification factor for the patients bones is calculated and stored for future use. The position of each of the bone implanted markers in the reference frame of the tracking system as is the position of the X-ray image marker 210 and the computer 122 also has access to the relative positioned orientation of the imaging plane relative to the position and orientation of the X-ray image marker 210.

In one embodiment, in order to allow a 3D image of the bone to be displayed, X-ray images of the bone from at least two different directions are captured. This can be achieved in a number of ways. One way of achieving this is using a single X-ray source and having the patient change the position of their bones, *e.g.* by rotating to approximately 90°. Another method would be to move the position of the X-ray sources, as illustrated by arrow 220 in Figure 5. The direction of arrow 220 is schematic only and in practice, the X-ray source would be rotated through approximately 90° around the bone or bones of interest. In a further embodiment, a second X-ray source 204'which bears a further marker 206'detectable by the tracking system 130. The first X-ray source 204 and second X-ray source 204' are preferably positioned to capture images at approximately 90° of each other. Method 250 will be described below using the example of a single X-ray source which is moved.

At step 256, with the X-ray source in a first position, an image of the patient's bones is captured and the position of the X-ray source in the reference frame of the tracking system is determined and captured. The captured image is correct using the linear magnification factor calculated previously and the corrected image is used subsequently. Then at step 258, the X-ray source is rotated through approximately 90° about the patient's body and a second image of the relevant body part is captured and again the position of the X-ray source in the reference frame of the tracking system is determining captured. The captured second image is correct using the linear magnification factor calculated previously and again the corrected second image is used subsequently. As there is a known relationship between the position of the X-ray source and the imaging plane of the X-ray detector, with the X-ray source in the first position, and as the position of the X-ray camera in the first position and in the second position in the reference frame of the tracking system are obtained from the track positions of the markers, it is possible to determine the relationship between the first captured X-ray image and the second captured X-ray image. Hence when a one of the images is registered with the bone position, the second image can also be registered or vice versa, as their fixed position relationship is known.

Method 250 is modified slightly in other embodiments of the imaging system 200. If the patient 214 moves, rather than the camera, then it is not necessary to track the position of the camera as there is a known fixed spacial relationship between the position and orientation of X-ray camera 204 and detector 208. Instead, the program uses the change in the orientation and position of the implanted markers (assuming the bone is otherwise in the same position) to determine the positional relationship between the first and second X-ray images. In the embodiment in which two separate X-ray camera sources 204, 204' are used, either the position of the first and second X-ray cameras can be detected or alternatively, there can be a known positional relationship between each of the cameras and the X-ray detector 208 which can be used to derive the positional relationship between the first and second X-ray images.

After the two X-ray images have been captured, irrespective of the method used, at step 260, a one of the X-ray images is brought into registration, in the reference frame of the computer control system, with the actual position of the bone in the reference frame of the computer control system. The method of registering the bone image and bone position will be described in greater detail below. Steps 254, 256 and 258 correspond generally to step 106 of method 100 and step 260 corresponds generally to step 108 of method 100. Step 260 does not necessarily include the generation of a registered image, but merely includes determining the relevant data to allow an image (either one of the captured images or an image derived from one of the captured images) to be mapped on to the position of the bone in the reference frame of the tracking system.

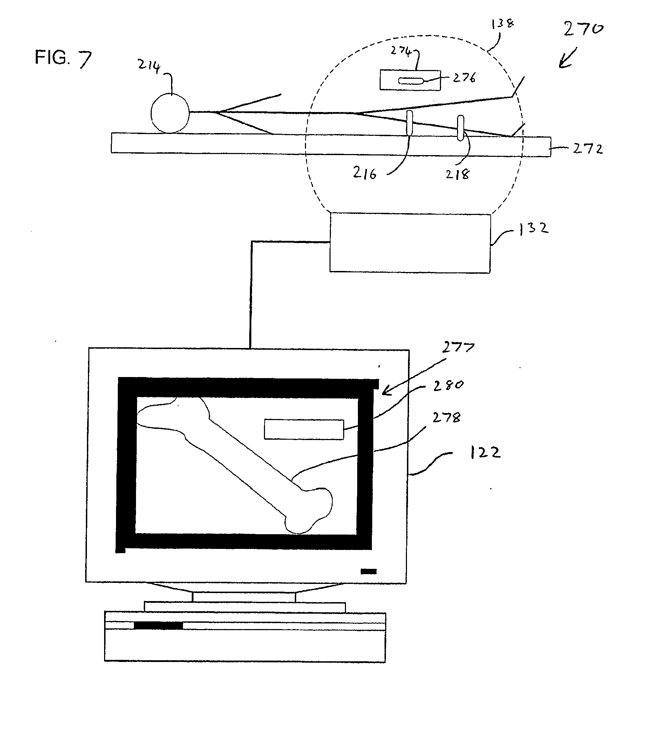

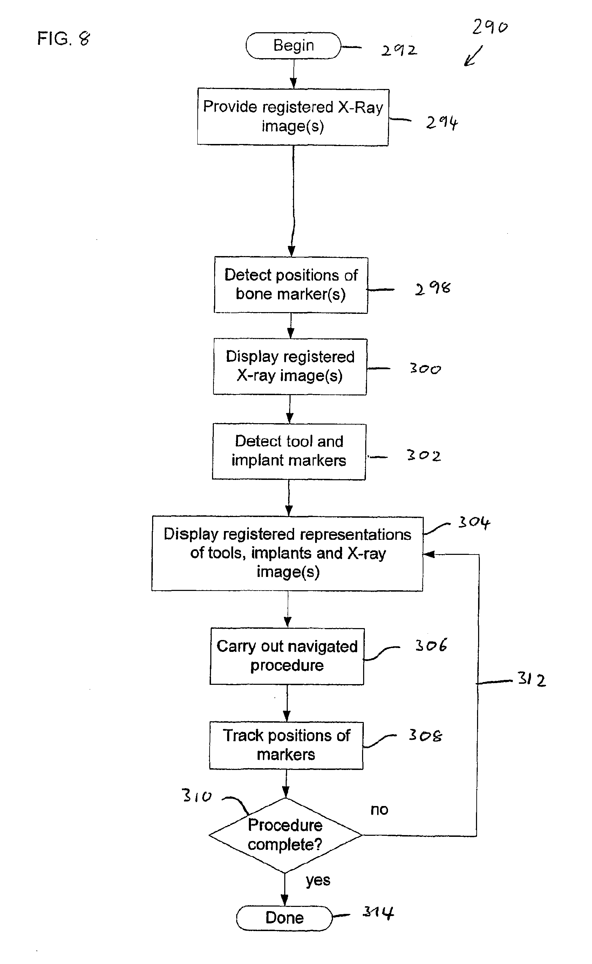

With reference to Figure 7 there is shown an operating room or operating theatre environment 270 and with reference to Figure 8 there is shown a method of using the registered image 290 corresponding generally to steps 110, 120 and 122 of method 100.

The operating room environment 270 includes the patient 214 and a patient support 272, e.g. an operating table. A tracking system 132 is provided with the patient located with the surgical site and trackable markers 216, 218 located generally within the tracking system magnetic field working volume 138. Also shown is computer control system 122 and a component 274 bearing a marker 276 detectable and trackable by the tracking system 132. The component 274 can be any component or device used in the surgical procedure, such as a surgical tool or instrument or an orthopaedic implant. Also shown is a visual display 277 including the registered image 278 on the display of computer control system 122. Computer control system 122 and tracking system 132 can be the same computer control system and tracking system used to capture the positional data previously, in which case the tracking system and computer control system are provided as a portable system. Or alternatively, they can be merely similar systems having the same tracking functionality but slightly different computer implemented control functionality.

In an embodiment in which the computer control system and tracking system are portable and the same as those used previously, then the computer control system 122 already has access to the data required to generate the registered image and can have had the digitised X-ray image data downloaded or stored therein. In an alternate embodiment, the data required to register the image and the image data can be supplied to computer system 122 on some form of computer readable medium or transmitted thereto over a network, such as a local area network or wide area network and including both wired and wireless networks.

Figure 8 shows a flowchart illustrating a method for carrying out a computer aided surgical procedure 290 utilising the registered image. The method begins at step 292 with the patient located in the operating room on the operating table with their knee joint within the tracking volume 138 of the tracking system 132. At step 294, computer system 122 makes the necessary calculations to map the bone image data on to the position of the bone in the reference frame of the tracking system.

After step 272, at step 298, the position of the implanted bone markers, 216, 218 is detected and the positions and orientations of the bone markers 216 and 218 are determined. Using the detected positions and orientations of bone markers 216, 218, at step 300 a three dimensional image of the patient's bones is rendered from the captured X-ray image data and the three dimensional image 278 is graphically displayed. As the image data is already registered with the positions of the actual body parts and as the position and orientations of the body parts has been determined in the operating room, it is possible to display a registered image of the bones without requiring any further registration procedures in the operating room. Also, as the orientations of the markers 216, 218 has been detected, it is possible to render, from the X-ray image data the appropriate three dimensional view of the bone from the direction corresponding to the orientation of the bones in the operating room.

After the registered image has been displayed at step 300, at step 302, the tracking system can detect the presence of marked tools, instruments and implants. Using the unique marker identifiers transmitted by the markers, the computer system 122 looks up graphics data and images associated with the marker identifiers. Some of the images to be displayed are corrected using the magnification factor determined previously to ensure that the representation of the tools and instruments are the appropriate size for the size of the bone images. After applying the magnification correction for each image for tools to be displayed and for navigation, at step 304, a graphical representation 180 of the physical component 274 is displayed in display 277 with the graphical representation being located at the position of the component and with the correct orientation of the component within the reference frame of the tracking system and relative to the position of the body part. Hence at step 304, a display 277 is provided showing a graphical representation of any tools, instruments and implants relative to the position of the registered bone image. At step 306, the surgical procedure can be started and carried out.

It will be appreciated that no invasive surgical steps have yet been carried out, but that already a registered image of the body part is available to the surgeon. For example step 306 can include planning the surgical procedure and then carrying out the surgical procedure using the navigated instruments and implants and using the displayed images 277 to guide the surgeon. At step 308, the positions of the markers within the working volume 138 is constantly tracked and if it is determined that the procedure is still ongoing and that tracking is still active at step 310, then the display is constantly updated, as represented by line 312 and a real time display of the current positions of the bone and instruments, tools and implants is rendered at step 304. When it is determined that the surgical procedure has been completed then the method can end at step 314.

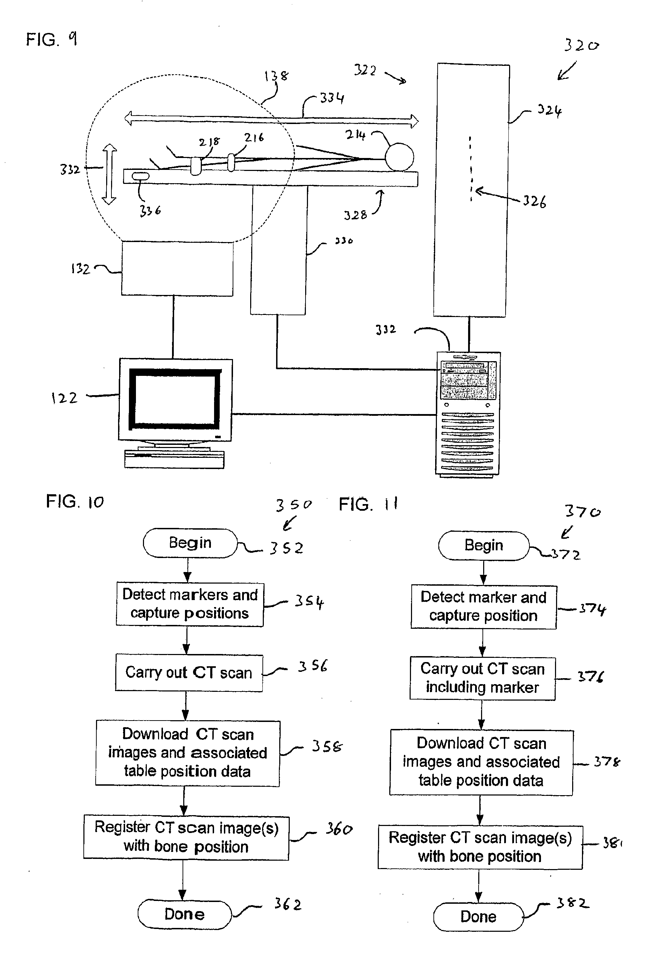

With reference to Figure 9 there is shown a further embodiment of a system 320 according to the invention including control computer 122, tracking system 132 and patient 214 having bone implanted markers 216, 218. In this embodiment, the imaging system is a CT scan system 322 including an image acquisition part 324 including an imaging plane 326. A patient support 328 is also included in the form of a table on a stand 330. A computer control system 322 generally controls the CT scan apparatus and stores the CT scan data. Computer control system 322 can control the position of table 328 including changing its height, as illustrated by arrow 332 and controlling the position of the patient within the image acquisition part 324, as illustrated by arrow 334 so as to collect a sequence of images of "slices" through the patient's bones.

There is a positional relationship between the imaging plane 326 and the position of table 328. Figure 10 illustrates a first method of using a first embodiment of system 320 in order to allow a registered image to be generated and Figure 11 illustrates a second method for using a second embodiment of system 320 to allow registered images to be generated.

In a first embodiment of system 320, table 328 includes a marker 336 detectable and trackable by the tracking system 132. The marker 336 is located at a known position relative to the table 328 and, as there is a known relationship between the position of the table and the imaging plane 326 at any table position, the position of the imaging plane 326 in the reference frame of the tracking system can be determined. The patient 214 is positioned on the table 328 and does not move during the CT scan procedure.

Figure 10 show a flowchart illustrating data processing operations carried out by computer control system 122 and implemented by a computer program 350. Program 350 is called and initiates at step 352. At step 354, the computer control system 122 determines the positions of markers 218, 216 and 336 in the reference frame of the tracking system. Computer 122 also determines the current positional relationship between table 328 and imaging plane 326 corresponding to the detected marker position 336. At step 356, the CT scan of the patient's knee is carried out. At step 358, the CT scan image data is downloaded from CT scan system control 332 together with data indicating the position of table 328 for each captured CT scan image. Then at step 360, at least a one of the scans of the patient's body part is registered with the position of the body part in the reference frame of the tracking system 132.

As there is a fixed spacial relationship between all of the images, once a one of the images has been brought into registration, the entire CT scan set of images can become registered. The bone markers 218, 216 indicate the position of the actual body part in the reference frame of the tracking system and using the position of table 328 in the reference frame of the tracking system, determined using the detected position of marker 336 and knowing the positional relationship between table 328 and image plane 326, the scanned images can be brought into registration with the bone positions as will be described in greater detail below.

Figure 11 shows a flowchart illustrating a computer implanted method allowing registered images to be generated using a second embodiment of system 320 and implemented by computer program 370. In this method, the table 328 of CT system 322 does not include marker 336. Rather, this method is based on capturing at least one image of the bone which includes an image or at least a part of the marker in the bone and determining the position of the marker at the time that the image is captured.

The table 328 is driven until a one of bone markers 218, 216 is located near the imaging plane 326. At step 374, the position of markers 216 and 218 is continuously determined and captured from tracking system 132 as, at step 376, the CT scan is carried out and the position of the markers 218, 216 is determined and captured for each of the scan images collected. It is preferred to capture a plurality of scan images including both the bone and marker although it is only necessary to capture at least one image showing both the bone and at least a part of one of the markers.

At step 378, the CT scan image data is downloaded from the CT system controller 3 32 to control computer 122 together with data indicating the position of the table. The position of the table is correlated with the marker positions such that each marker position is correlated with a corresponding CT scan image.

Bone markers 216, 218 determine the position and orientation of the bone in the reference frame of the tracking system. At least a one of the CT scan images also shows at least a part of one of the markers and therefore the position of that image in the reference frame of the tracking system is known. Then at step 380, the CT image including the markers is brought into registration with the position of the bone in the reference frame of the tracking system thereby registering the entire set of CT scan images. It is not necess ary to actually register the images at step 380 but merely to determine the mapping vector or translation required in order to allow the registered image to be generated subsequently. Hence at step 380 and similarly at step 360, all that is determined is the mapping that determines the relationship between the position of the captured images in the reference frame of the scanning system and the bone position in the reference frame of the tracking system.

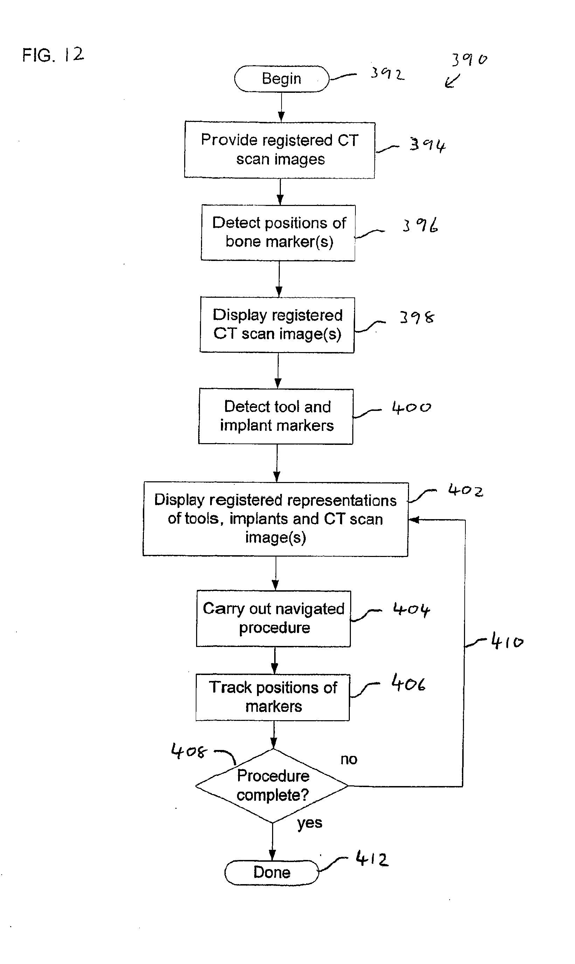

With reference to Figure 12 there is shown a flowchart illustrating a method 390 in which a registered image obtained from the system 320 shown in Figure 9 can be used. Method 390 is generally similar to method 290 as shown in Figure 8 and so will not be described in great detail. In method 390, instead of displaying X-ray images, CT scan images are displayed and again the displayed CT scan images can either be the actual images captured or are preferably three dimensional images derived from the captured CT scan images which reflect the appearance of the bone with the patient in the orientation on the operating room table as determined by the position and orientation captured from the bone markers.

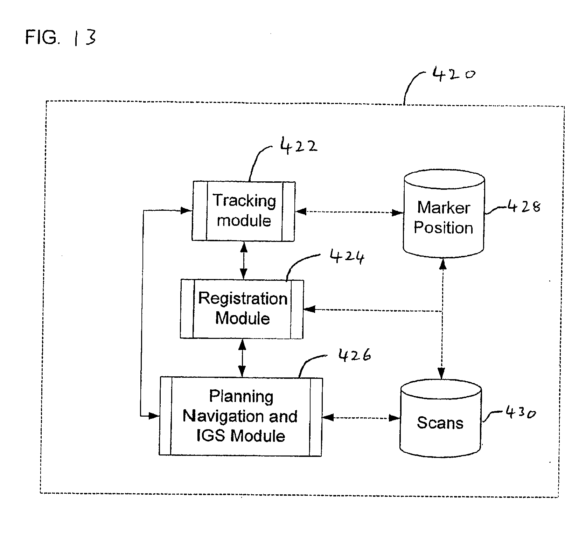

With reference to Figure 13, there is shown a schematic representation of a software architecture 420 for the computer control system 122. Software architecture 420 includes a number of conceptual parts which in practice can be implemented by separate programs or a single program with different parts interacting with each other to provide the desired functionality. Architecture 420 should be understood at a conceptual level and is not intended to limit the invention, e.g. to three separate programs only.

Computer control system 122 includes an operating system under which various application programs forming part of architecture 420 execute. A tracking program or programs 422 processes signals and/or data received from the tracking system 132 and provides marker IDs, positions and orientation data to a registration program 424 and/or to a computer aided surgery program or programs 426. Where different computer control system 122 and tracking systems 132 are used for the image registration and surgical procedure, then the image registration system uses tracking program 432 and registration program 424 only. The computer aided surgery module then uses tracking module 422 and computer aided surgery module 426. When a single computer control system 122 ands tracking system 132 are used to implement both the image registration and computer aided surgery procedures, then the tracking, registration and computer aided surgery modules are all provided.

As illustrated in Figure 13, a database 428 for storing data relating to and derived from the trackable markers can be provided. Database 428 can store various data items, including patient identified data items, marker ID data items, which uniquely identify each marker. Other data associated with the markers can also be stored in marker database 428, including the position of a marker relative to a part of an imaging system, such as the position of marker 210 relative to the imaging plane and marker 336 relative to the table. Also, the implant, instrument or tool associated with a marker can also be identified from database 428. A database 430 is also provided which stores the "raw" X-ray or CT scan data and which is used in rendering the three dimensional images of the body parts for display during the computer aided surgical procedure. Other data associated with the body scan images can also be stored in database 430, including the relative position of a scan to another scan so as to allow multiple images to be registered concurrently once a one of the images has been registered.

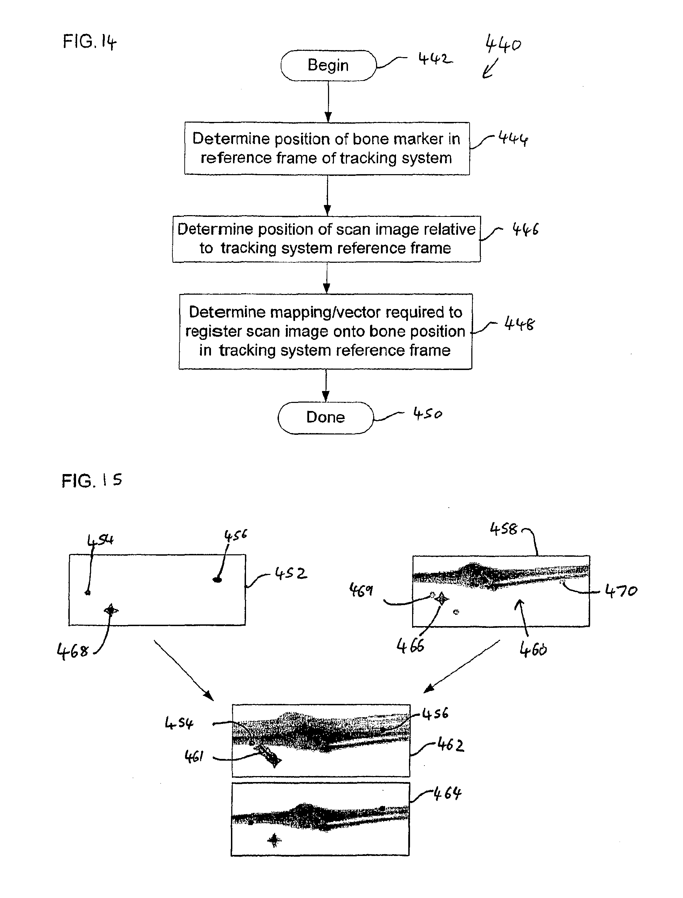

The functioning of registration module 424 will be described in greater detail with reference to Figures 14 and 15 below. Computer aided surgery module 426 includes a number of applications, including applications providing surgical planning functionality, instrument, tool and implant navigational functionality and image guided surgery functionality. The computer aided surgery module 426 interacts with tracking module 422 to obtain marker IDs and current marker position and orientation data, as well as bone position and orientation data so as to allow the real time display of a representation of the registered image and representations of any tools and/or implants as illustrated in Figure 7 and corresponding generally to method steps 304 and 402.

Registration program 424 can have access both to the marker database 428 and scan database 430 and can either store data required for generating a registered image in the scan database or supplied to the computer aided surgery module 426 so as to allow the registered image to be generated. Registration program 424 includes instructions for carrying out methods 250, 350 and 370.

With reference to Figures 14 and 15, the processes involved in registering the image data with the position of the body part (corresponding generally to method steps 260, 360 and 380) will now be described with reference to Figures 14 and 15. Figure 14 shows a flowchart illustrating a method for allowing a registered image to be generated implemented by computer program 440. The registration process 440 is called and begins at step 442. At step 444, the position of at least one bone marker is determined in the reference frame of the tracking system. Figure 15 provides a graphical representation of the operations carried out by registration process 440. In Figure 15, a graphical representation of the position of bone marker 216, position 454 and of bone marker 318, position 456, in the reference frame of the tracking system 452 is determined. At step 446, the position of the image 460 in the reference frame 458 of the tracking system is determined. Then at step 448, a vector 461 in the reference frame of the tracking system which maps the bone image 460 on to the position of the bone, as determined by the marker positions 554, 556 is determined and can subsequently be used to generate the registered image 464 which completes the registration procedure at step 450. In practice, vector 461 can be provided by a transformation matrix which is applied to the image data.

The mapping 461 which is required can be determined in a number of ways, depending on the system used. For example when marker 336 is used in CT scan table 328, then point 468 corresponds to the detected position of marker 336 in the reference frame of the tracking system and position 466 corresponds to the position of the marker relative to the image. The mapping vector 461 is therefore the vector required to map position 466 on to position 468 in the reference frame of the tracking system. In the embodiment in which image 460 also includes an image of a part of the marker, then point 466 corresponds to the position of the marker in the image and the appropriate mapping is that vector which maps the position of the marker 466 in the image on to the detected position of the marker 468 in the reference frame of the tracking system at the time that the image 460 was captured.

In the X-ray system embodiment 200 point 469 corresponds to the position of bone implant 216 in the imaging plane of the X-ray detector and point 470 corresponds to the position of bone implant 218 in the imaging plane of the X-ray detector. Point 454 corresponds to the position of bone marker 216 and point 456 corresponds to the position of bone marker 218 in the reference frame of the tracking system. Using marker 210, the position of the image 460 in the reference frame of the tracking system is known and so the positions of the bone markers relative to the image, 469, 470 are derivable therefrom. Therefore step 448 corresponds to determining the vector required to map points 469 and 470 in image 460 on to the detected bone positions 454 and 456 so as to generate the registered image 464.

In another embodiment, the bone markers 216, 218 are not imaged in the x-ray (so not seen on 458 as 469 + 470) but are located in the same bone as is shown in the image. The image can still be registered as the position of the markers and hence the bone is registered in the computer control system even though not shown in the image, and as there is a known fixed positional relationship between the markers and the parts of bone shown in the image.

Generally, embodiments of the present invention employ various processes involving data stored in or transferred through one or more computer systems. Embodiments of the present invention also relate to an apparatus for performing these operations. This apparatus may be specially constructed for the required purposes, or it may be a general-purpose computer selectively activated or reconfigured by a computer program and/or data structure stored in the computer. The processes presented herein are not inherently related to any particular computer or other apparatus. In particular, various general-purpose machines may be used with programs written in accordance with the teachings herein, or it may be more convenient to construct a more specialized apparatus to perform the required method steps. A particular structure for a variety of these machines will appear from the description given below.

In addition, embodiments of the present invention relate to computer readable media or computer program products that include program instructions and/or data (including data structures) for performing various computer-implemented operations. Examples of computer-readable media include, but are not limited to, magnetic media such as hard disks, floppy disks, and magnetic tape; optical media such as CD-ROM disks; magnetooptical media; semiconductor memory devices, and hardware devices that are specially configured to store and perform program instructions, such as read-only memory devices (ROM) and random access memory (RAM). The data and program instructions of this invention may also be embodied on a carrier wave or other transport medium. Examples of program instructions include both machine code, such as produced by a compiler, and files containing higher level code that may be executed by the computer using an interpreter.

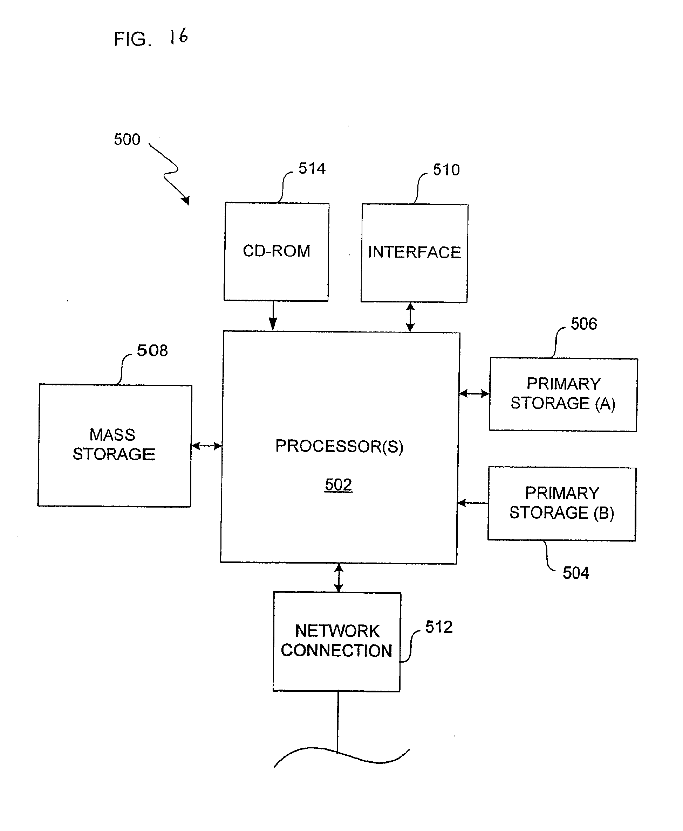

Figure 16 illustrates a typical computer system that, when appropriately configured or designed, can serve as an image analysis apparatus of this invention. The computer system 500 includes any number of processors 502 (also referred to as central processing units, or CPUs) that are coupled to storage devices including primary storage 506 (typically a random access memory, or RAM), primary storage 504 (typically a read only memory, or ROM). CPU 502 may be of various types including microcontrollers and microprocessors such as programmable devices (e.g., CPLDs and FPGAs) and unprogrammable devices such as gate array ASICs or general purpose microprocessors. As is well known in the art, primary storage 504 acts to transfer data and instructions uni-directionally to the CPU and primary storage 506 is used typically to transfer data and instructions in a bi-directional manner. Both of these primary storage devices may include any suitable computer-readable media such as those described above. A mass storage device 508 is also coupled bi-directionally to CPU 502 and provides additional data storage capacity and may include any of the computer-readable media described above. Mass storage device 508 may be used to store programs, data and the like and is typically a secondary storage medium such as a hard disk. It will be appreciated that the information retained within the mass storage device 508, may, in appropriate cases, be incorporated in standard fashion as part of primary storage 506 as virtual memory. A specific mass storage device such as a CD-ROM 514 may also pass data uni-directionally to the CPU.

CPU 502 is also coupled to an interface 510 that connects to one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 502 optionally may be coupled to an external device such as a database or a computer or telecommunications network using an external connection as shown generally at 512. With such a connection, it is contemplated that the CPU might receive information from the network, or might output information to the network in the course of performing the method steps described herein.

Although the above has generally described the present invention according to specific processes and apparatus, the present invention has a much broader range of applicability. In particular, aspects of the present invention is not limited to any particular kind of orthopaedic performance and can be applied to virtually any joint or body structure, whether including an implant or implants or not, with relatively moving bones where an image of the body part in a form in which it can be registered is desired. Thus, in some embodiments, the techniques of the present invention could provide a registered image for use surgery not involving implants, e.g. a cruciate ligament operation, as well as implant replated surgical techniques. One of ordinary skill in the art would recognize other variants, modifications and alternatives in light of the foregoing discussion.

It will also be appreciated that the invention is not limited to the specific combinations of structural features, data processing operations, data structures or sequences of method steps described and that, unless the context requires otherwise, the foregoing can be altered, varied and modified. For example different combinations of structural features can be used and features described with reference to one embodiment can be combined with other features described with reference to other embodiments. Similarly the sequence of the methods step can be altered and various actions can be combined into a single method step and some methods steps can be carried out as a plurality of individual steps. Also some of the structures are schematically illustrated separately, or as comprising particular combinations of features, for the sake of clarity of explanation only and various of the structures can be combined or integrated together or different features assigned to other structures.

## Claims

1. A method (250, 350, 370) for generating and displaying a registered image of a body part of a patient for use in a computer aided surgical procedure, the method comprising:
capturing (256, 356, 376) at least a first image of the body part using an imaging system (202, 322) at a first location, wherein a first marker (210, 336) detectable by a tracking system (132) having a reference frame is attached to part of the imaging system;
detecting (254, 354, 374) the position of a second marker (216, 218), which has been attached to the body part prior to any surgical steps of the surgical procedure, while the at least a first image is being captured using the tracking system;
detecting (354, 354, 374) the position of the first marker (210, 336) while the at least a first image is being captured using the tracking system;
obtaining (446) an indication of the position of the first image in the reference frame of the tracking system using the detected position of the first marker and a known positional relationship between the first marker and an image plane of the imaging system;
determining (448) a mapping (461) to bring the first image (460) into registration with the position of the body part in the reference frame using the detected position (444) of the second marker in the reference frame;
detecting (298, 386) the position of the second marker while the body part is in an operating theatre, being a different location to the first location, using either the tracking system which has been moved to the operating theatre from the first location, or a second tracking system and determining the position of the body part in the reference frame of the tracking system or second tracking system; and
displaying (300, 398) the registered image by using the mapping to register the first image with the detected position of the body part in the operating theatre while the body part is in the operating theatre.

2. The method as claimed in claim 1, wherein the first image (460) includes the second marker (466) and at least a part of the body part, and wherein the position of the second marker in the first image is used to determine the mapping (461).

3. The method as claimed in claim 2, wherein the surgical procedure (290) is an orthopaedic procedure.

4. The method as claimed in any preceding claim, wherein the second marker (216, 218) is wirelessly detectable at radio frequencies by the tracking system.

5. The method as claimed in any preceding claim, wherein the imaging system (202) is an X-ray system.

6. The method as claimed in claim 5, wherein the position of the second marker is detected with the patient (214) standing.

7. The method as claimed in any preceding claim, wherein the second marker (216, 218) is wirelessly tracked using a magnetic tracking system (132).

8. The method as claimed in any of claims 9 to 11, wherein the first marker (210) is attached to an X-ray detector (208) and the method includes determining the position of the X-ray detector (280) in the reference frame of the tracking system (132).

9. The method of any of claims 5 to 8, and further comprising capturing a second image of the body part using the X-ray system (202), and wherein the second image is in a second direction different to a first direction in which the first image was captured.

10. The method as claimed in claim 9, wherein capturing the second image includes moving the patient (214) relative to the X-ray system (202).

11. The method as claimed in claim 10, wherein capturing the second image includes moving an X-ray source (204) relative to the patient (214) and the method further comprises using the tracking system (132) to determine the position of the X-ray source (204) in the reference frame of the tracking system when the second image is captured.

12. The method as claimed in claim 9, wherein the first image is captured using a first X-ray source (204) and wherein capturing the second image includes using a second X-ray source (204') at a second position which is different to a first position of the first X-ray source.

13. The method as claimed in claim 9 and further comprising generating a three dimensional image of the body part from the first and second images.

14. The method as claimed in claim 5 and further comprising determining the distance (d) between the body part (214) and an imaging plane of an X-ray detector (208) along a direction perpendicular to the plane of the imaging plan and using the distance to correct the first image captured by the X-ray detector.

15. The method as claimed in any of claims 1 to 4, wherein the imaging system is a CT scan (322) or an MR scan system.

16. The method as claimed in claim 15, wherein the body part of the patient (214) is located on a patient support (328) part of the imaging system when the first image is captured and further comprising determining the position of the patient support part (328) in the reference frame of the tracking system by tracking the first marker (336) using the tracking system.

17. The method as claimed in claim 16, and wherein the first marker (336) is mounted on the patient support part (328).

18. The method as claimed in claim 15, wherein the body part of the patient is located on a patient support part of the imaging system when the first image is captured and further comprising determining the position of the imaging plane (326) of the imaging system relative to the position of the patient support part (328) from the tracked position of the first marker (336).

19. The method as claimed in claim 15, wherein the first image includes the second marker and at least a part of the body part.

20. The method as claimed in any preceding claim and wherein:
a third marker (218) detectable by the tracking system has been attached to a further body part prior to any surgical steps of the image guided surgical procedure; and the method further comprising detecting the position of the third marker (218) in the reference frame.

21. The method as claimed in claim 20, and wherein mapping the first image into registration with the position of the body part includes using the position of the third marker in the reference frame.

22. A system (130, 320) for generating and displaying a registered image of a body part of a patient (214) for use in an image guided surgical procedure, the system including:
a tracking system (132) for detecting markers and determining the position of the markers in a reference frame of the tracking system;
an imaging system (202, 322) at a first location for capturing at least a first image of the body part, wherein a first marker (210, 336) detectable by the tracking system is attached to a part of the imaging system;
a second marker (216, 218) attachable to the body part of the patient (218) and detectable by the tracking system; and
a computer control system (122) configured to:
cause the imaging system to capture (256, 356, 376) at least a first image of the body part;
cause the tracking system to detect (254, 354, 374) the position of the second marker (216, 218) when attached to the body part while the at least first image is being captured at the first location;
cause the tracking system to detect (254, 354, 374) the position of the first marker (210, 336) while the at least first image is being captured;
obtain an indication of the position of the first image in the reference frame of the tracking system using the detected position of the first marker and a known positional relationship between the first marker (210, 336) and an image plane (326) of the imaging system;
determine (448) how to map the first image into registration with the position of the body part in the reference frame using the detected position (444) of the second marker in the reference frame;
cause either the tracking system when moved to an operating theatre, or a further tracking system in the operating theatre, to detect (298, 386) the position of the second marker while the body part is in the operating theatre, being a different location to the first location, and determine the position of the body part in the reference frame of the tracking system or further tracking system; and
display (300, 398) the registered image by using the mapping to register the first image with the detected position of the body part in the operating theatre.

23. A system as claimed in claim 22, wherein the second marker (216, 218) is wirelessly detectable by the tracking system (132).

24. A system as claimed in claim 23, wherein the second marker (216, 218) is wirelessly detectable by the tracking system (132) at radio frequencies.

25. A system as claimed in any of claims 23 to 25, wherein the second marker (216, 218) is percutaneously implantable in a bone of the patient.

26. A system as claimed in any of claims 23 to 26, wherein the imaging system (202) is an X-ray imaging system including an X-ray source (204) and an X-ray detector (208) having the imaging plane.

27. A system as claimed in claim 26, wherein the first marker (210) is mounted on the X-ray detector (208).

28. A system as claimed in claim 26 or 27, and further comprising a third marker (206) detectable by the tracking system, and wherein the third marker is mounted on the X-ray source (204) and wherein the X-ray source (204) is movable relative to the X-ray detector (208).

29. A system as claimed in claim 26 or 27, and further comprising:
a fourth marker (206') detectable by the tracking system; and
a further X-ray source (204'), and wherein the fourth marker is mounted on the further X-ray source.

30. A system as claimed in any of claims 26 to 29, wherein each marker is a magnetically detectable and trackable marker.

31. A system as claimed in any of claims 26 to 30, wherein the computer control system (122) is further configured to determine a separation (d) between the second marker (216, 218) and an imaging plane of the X-ray detector (208) and to use the separation to correct the magnification of the first image.

32. A system as claimed in any of claims 22 to 27, wherein the imaging system is a CT scan or MR scan imaging system (322).

33. A system as claimed in claim 32, wherein the imaging system includes a patient support part (328) and the first marker (336) is mounted on the patient support part.

34. A system as claimed in claim 33, wherein the computer control system (122) is further configured to determine the position of the patient (214) when the first image includes the second marker and at least a part of the body part.

35. A system as claimed in claim 33, wherein the imaging system includes a patient support part (328) and wherein the computer control system (122) determines the position of an imaging plane (326) of the imaging system relative to the patient support part (328).

36. A system as claimed in any of claims 26 to 35, and further comprising a third marker (218) attachable to a further body part of the patient (214) and detectable by the tracking system (132).

37. A computer implemented method (440) for generating and displaying a registered image of a body part of a patient (214) bearing a second marker (216, 218) detectable by a tracking system (132) having a reference frame, the registered image being for use in an image guided surgical procedure and being generated prior to any surgical steps of the image guided surgical procedure, the method comprising:
determining (444) the position of the second marker (454, 456, 468) in the reference frame (452), from the position of the second marker tracked by the tracking system while a first image of the body part was being captured by an imaging system at a first location;
determining (446) the position of a first image (460) of the body part in the reference frame (458) of the tracking system, from the position of a first marker attached to a part of the imaging system and tracked by the tracking system while the first image of the body part was being captured and a known positional relationship between the first marker and an image plane of the imaging system;
determining (448) a mapping (461) to bring the first image (460) into registration with the position of the body part in the reference frame using the detected position of the second marker in the reference frame;
determining (298, 396) the position of the body part in the reference frame of the tracking system or a second tracking system from the position of the second marker while the body part is in an operating theatre, being a location different to the first location, and tracked by either the tracking system which has been moved to the operating theatre or a second tracking system in the operating theatre; and
displaying (300, 398) the registered image by using the mapping to register the first image with the detected position of the body part in the operating theatre.

38. A method as claimed in claim 37, wherein the first image includes a further body part of the patient bearing a third marker detectable by the tracking system, and the method further comprising determining the position of the third marker (456) in the reference frame.

39. A method as claimed in claim 37, wherein determining the position of the first image includes determining the position of the imaging plane (326) of the imaging system relative to a patient support part (328) of the imaging system.

40. A method as claimed in claim 37, 38 or 39, wherein determining the position of a first image of the body part in the reference frame includes determining the position of an image (466) of at least a part of the second marker in the first image (460).

41. A computer readable medium bearing computer program code executable by a data processing device to provide a method as claimed in any of claims 38 to 40.

## Patentansprüche

1. Verfahren (250, 350, 370) zum Erzeugen und Anzeigen eines ausgerichteten Bildes eines Körperteils eines Patienten zum Verwenden bei einem computer-unterstützen chirurgischen Eingriff, wobei das Verfahren Folgendes umfasst:
Erfassen (256, 356, 376) zumindest eines ersten Bilds des Körperteils, wobei ein abbildendes System (202, 322) an einem ersten Ort verwendet wird, wobei ein erster Markierer (210, 336), der von einem Verfolgungssystem (132), das einen Bezugsrahmen aufweist, detektierbar ist, an einem Teil des abbildenden Systems angebracht ist,
Detektieren (254, 354, 374) der Position eines zweiten Markierers (216, 218), der vor chirurgischen Schritten des chirurgischen Eingriffs an dem Körperteil angebracht worden ist, während das zumindest erste Bild erfasst wird, wobei das Verfolgungssystem verwendet wird,
Detektieren (354, 354, 374) der Position des ersten Markierers (210, 336), während das zumindest erste Bild erfasst wird, wobei das Verfolgungssystem verwendet wird,
Ermitteln (446) einer Angabe der Position des ersten Bildes in dem Bezugsrahmen des Verfolgungssystems, wobei die detektierte Position des ersten Markierers und eine bekannte Positionsbeziehung zwischen dem ersten Markierer und einer Bildebene des abbildenden Systems verwendet werden,
Bestimmen (448) einer Abbildung (461), um das erste Bild (460) in eine Ausrichtung mit der Position des Körperteils in dem Bezugsrahmen zu bringen, wobei die detektierte Position (444) des zweiten Markierers in dem Bezugsrahmen verwendet wird,
Detektieren (298, 386) der Position des zweiten Markierers, während sich der Körperteil in einem Operationssaal befindet, wobei es sich um einen anderen Ort als den ersten Ort handelt, wobei entweder das Verfolgungssystem, das von dem ersten Ort zu dem Operationssaal bewegt worden ist, oder ein zweites Verfolgungssystem verwendet wird, und Bestimmen der Position des Körperteils in dem Bezugsrahmen des Verfolgungssystems oder des zweiten Verfolgungssystems, und
Anzeigen (300, 398) des ausgerichtenen Bildes, indem die Abbildung dazu verwendet wird, dass erste Bild mit der detektierten Position des Körperteils in dem Operationssaal auszurichten, während sich der Körperteil in dem Operationssaal befindet.

2. Verfahren gemäß Anspruch 1, bei dem das erste Bild (460) den zweiten Markierer (466) und zumindest einen Teil des Körperteils enthält, und bei dem die Position des zweiten Markierers in dem ersten Bild dazu verwendet wird, die Abbildung (461) zu bestimmen.

3. Verfahren gemäß Anspruch 2, bei dem der chirurgische Eingriff (290) ein orthopädischer Eingriff ist.

4. Verfahren gemäß einem der vorhergehenden Ansprüche, bei dem der zweite Markierer (216, 218) bei Funkfrequenzen drahtlos von dem Verfolgungssystem detektierbar ist.

5. Verfahren gemäß einem der vorhergehenden Ansprüche, bei dem das abbildende System (202) ein Röntgensystem ist.

6. Verfahren gemäß Anspruch 5, bei dem die Position des zweiten Markierers detektiert wird, wobei der Patient (214) steht.

7. Verfahren gemäß einem der vorhergehenden Ansprüche, bei dem der zweite Markierer (216, 218) drahtlos verfolgt wird, wobei ein magnetisches Verfolgungssystem (132) verwendet wird.

8. Verfahren gemäß einem der Ansprüche 9 bis 11, bei dem der erste Markierer (210) an einem Röntgendetektor (208) angebracht ist, und bei dem das Verfahren ein Bestimmen der Position des Röntgendetektors (280) in dem Bezugsrahmen des Verfolgungssystems (132) enthält.

9. Verfahren nach einem der Ansprüche 5 bis 8, das ferner ein Erfassen eines zweiten Bildes des Körperteils umfasst, wobei das Röntgensystem (202) verwendet wird, und bei dem das zweite Bild in eine zweite Richtung ist, die von einer ersten Richtung, in die das erste Bild erfasst worden ist, verschieden ist.

10. Verfahren gemäß Anspruch 9, bei dem das Erfassen des zweiten Bildes ein Bewegen des Patienten (214) relativ zu dem Röntgensystem (202) aufweist.

11. Verfahren gemäß Anspruch 10, bei dem das Erfassen des zweiten Bildes ein Bewegen einer Röntgenquelle (204) relativ zu dem Patienten (214) aufweist, und bei dem das Verfahren ferner ein Verwenden des Verfolgungssystems (132) umfasst, um die Position der Röntgenquelle (204) in dem Bezugsrahmen des Verfolgungssystems zu bestimmen, wenn das zweiten Bild erfasst wird.

12. Verfahren gemäß Anspruch 9, bei dem das erste Bild erfasst wird, wobei eine erste Röntgenquelle (204) verwendet wird, und bei dem das Erfassen des zweiten Bildes ein Verwenden einer zweiten Röntgenquelle (204') an einer zweiten Position, die von einer ersten Position der ersten Röntgenquelle verschieden ist, aufweist.

13. Verfahren gemäß Anspruch 9, das ferner ein Erzeugen eines dreidimensionalen Bildes des Körperteils aus dem ersten und dem zweiten Bild umfasst.

14. Verfahren gemäß Anspruch 5, das ferner ein Bestimmen des Abstands (d) zwischen dem Körperteil (214) und einer Bildebene eines Röntgendetektors (208) entlang einer Richtung senkrecht zu der Ebene der Bildebene umfasst, wobei der Abstand dazu verwendet wird, das erste von dem Röntgendetektor erfasste Bild zu korrigieren.

15. Verfahren gemäß einem der Ansprüche 1 bis 4, bei dem das abbildende System ein CT-Scan- (322) oder ein MR-Scan-System ist.

16. Verfahren gemäß Anspruch 15, bei dem sich der Körperteil des Patienten (214) auf einem Patientenstützteil (328) des abbildenden Systems befindet, wenn das erste Bild erfasst wird, und das ferner ein Bestimmen der Position des Patientenstützteils (328) in dem Bezugsrahmen des Verfolgungssystems durch ein Verfolgen des ersten Markierers (336) umfasst, wobei das Verfolgungssystem verwendet wird.

17. Verfahren gemäß Anspruch 16, bei dem der erste Markierer (336) an dem Patientenstützteil (328) montiert ist.

18. Verfahren gemäß Anspruch 15, bei dem sich der Körperteil des Patienten auf einem Patientenstützteil des abbildenden Systems befindet, wenn das erste Bild erfasst wird, und das ferner ein Bestimmen der Position der Bildebene (326) des abbildenden Sys-tems relativ zu der Position des Patientenstützteils (328) aus der verfolgten Position des ersten Markierers (336) umfasst.

19. Verfahren gemäß Anspruch 15, bei dem das erste Bild den zweiten Markierer und zumindest einen Teil des Körperteils enthält.

20. Verfahren gemäß einem der vorhergehenden Ansprüche, bei dem ein dritter Markierer (218), der von dem Verfolgungssystem detektierbar ist, vor chirurgischen Schritten des bildgeführten chirurgischen Eingriffs an einem weiteren Körperteil angebracht worden ist, und bei dem das Verfahren ferner ein Detektieren der Position des dritten Markierers (218) in dem Bezugsrahmen umfasst.

21. Verfahren gemäß Anspruch 20, bei dem das Abbilden des ersten Bildes in die Ausrichtung mit der Position des Körperteils ein Verwenden der Position des dritten Markierers in dem Bezugsrahmen aufweist.

22. System (130, 320) zum Erzeugen und Anzeigen eines ausgerichteten Bildes eines Körperteils eines Patienten (214) zum Verwenden bei einem bildgeführten chirurgischen Eingriff, wobei das System Folgendes enthält:
ein Verfolgungssystem (132) zum Detektieren von Markierern und zum Bestimmen der Position der Markierer in einem Bezugsrahmen des Verfolgungssystems;
ein abbildendes System (202, 322) an einem ersten Ort zum Erfassen zumindest eines ersten Bildes des Körperteils, wobei ein erster Markierer (210, 336), der von dem Verfolgungssystem detektierbar ist, an einem Teil des abbildenden Systems angebracht ist,
einen zweiten Markierer (216, 218), der an dem Körperteil des Patienten (218) anbringbar ist und von dem Verfolgungssystem detektierbar ist, und
ein Computersteuerungssystem (122), das zu Folgendem konfiguriert ist:
Bewirken, dass das abbildende System zumindest ein erstes Bild des Körperteils erfasst (256, 356, 376),
Bewirken, dass das Verfolgungssystem die Position des zweiten Markierers (216, 218) detektiert (254, 354, 374), wenn er an dem Körperteil angebracht ist, während das zumindest erste Bild an dem ersten Ort erfasst wird,
Bewirken, dass das Verfolgungssystem die Position des ersten Markierers (210, 336) detektiert (254, 354, 374), während das zumindest erste Bild erfasst wird,
Ermitteln einer Angabe der Position des ersten Bildes in dem Bezugsrahmen des Verfolgungssystems, wobei die detektierte Position des ersten Markierers und eine bekannte Positionsbeziehung zwischen dem ersten Markierer (210, 336) und einer Bildebene (326) des Bildsystems verwendet werden,
Bestimmen (448), wie das erste Bild in eine Ausrichtung mit der Position des Körperteils in dem Bezugsrahmen abgebildet wird, wobei die detektierte Position (444) des zweiten Markierers in dem Bezugsrahmen verwendet wird,
Bewirken, dass entweder das Verfolgungssystem, wenn es zu einem Operationssaal bewegt worden ist, oder ein weiteres Verfolgungssystem in dem Operationssaal die Position des zweiten Markierers detektiert (298, 386), während sich der Körperteil in dem Operationssaal befindet, wobei es sich um einen anderen Ort als den ersten Ort handelt, und die Position des Körperteils in dem Bezugsrahmen des Verfolgungssystems oder des weiteren Verfolgungssystems bestimmt, und
Anzeigen (300, 398) des ausgerichteten Bildes, indem die Abbildung dazu verwendet wird, das erste Bild mit der detektierten Position des Körperteils in dem Operationssaal auszurichten.

23. System gemäß Anspruch 22, bei dem der zweite Markierer (216, 218) von dem Verfolgungssystem (132) drahtlos detektierbar ist.

24. System gemäß Anspruch 23, bei dem der zweite Markierer (216, 218) von dem Verfolgungssystem (132) bei Funkfrequenzen drahtlos detektierbar ist.

25. System gemäß einem der Ansprüche 23 bis 25, bei dem der zweite Markierer (216, 218) perkutan in einen Knochen des Patienten implantierbar ist.

26. System gemäß einem der Ansprüche 23 bis 26, bei dem das abbildende System (202) ein Röntgenabbildungssystem, das eine Röntgenquelle (204) und einen Röntgendetektor (208) mit der Bildebene enthält, ist.

27. System gemäß Anspruch 26, bei dem der erste Markierer (210) auf dem Röntgendetektor (208) montiert ist.

28. System gemäß Anspruch 26 oder 27, das ferner einen dritten Markierer (206), der von dem Verfolgungssystem detektierbar ist, umfasst, wobei der dritte Markierer auf der Röntgenquelle (204) montiert ist, und wobei die Röntgenquelle (204) relativ zu dem Röntgendetektor (208) bewegbar ist.

29. System gemäß Anspruch 26 oder 27, das ferner Folgendes umfasst:
einen vierten Markierer (206'), der von dem Verfolgungssystem detektierbar ist, und
eine weitere Röntgenquelle (204'), wobei der vierte Markierer auf der weiteren Röntgenquelle montiert ist.

30. System gemäß einem der Ansprüche 26 bis 29, bei dem jeder Markierer ein magnetisch detektierbarer und verfolgbarer Markierer ist.

31. System gemäß einem der Ansprüche 26 bis 30, bei dem das Computersteuerungssystem (122) ferner dazu konfiguriert ist, einen Abstand (d) zwischen dem zweiten Markierer (216, 218) und einer Bildebene des Röntgendetektors (208) zu bestimmen und den Abstand dazu zu verwenden, die Vergrößerung des ersten Bildes zu korrigieren.

32. System gemäß einem der Ansprüche 22 bis 27, bei dem das abbildende System ein CT-Scan- oder ein MR-Scan-Abbildungssystem (322) ist.

33. System gemäß Anspruch 32, bei dem das abbildende System einen Patientenstützteil (328) enthält, und bei dem der erste Markierer (338) auf dem Patientenstützteil montiert ist.

34. System gemäß Anspruch 33, bei dem das Computersteuerungssystem (122) ferner dazu konfiguriert ist, die Position des Patienten (214) zu bestimmen, wenn das erste Bild den zweiten Markierer und zumindest einen Teil des Körperteils enthält.

35. System gemäß Anspruch 33, bei dem das abbildende System einen Patientenstützteil (328) enthält, und bei dem das Computersteuerungssystem (122) die Position einer Bildebene (326) des abbildenden Systems relativ zu dem Patientenstützteil (328) bestimmt.

36. System gemäß einem der Ansprüche 26 bis 35, das ferner einen dritten Markierer (218), der an einem weiteren Körperteil des Patienten (214) anbringbar ist und von dem Verfolgungssystem (132) detektierbar ist, umfasst.

37. Computerimplementiertes Verfahren (440) zum Erzeugen und Anzeigen eines ausgerichteten Bildes eines Körperteils eines Patienten (214), der einen zweiten Markierer (216, 218), der von einem Verfolgungssystem (132) mit einem Bezugsrahmen detektierbar ist, trägt, wobei das ausgerichtete Bild dem Verwenden bei einem bildgeführten chirurgischen Eingriff dient und vor chirurgischen Schritten des bildgeführten chirurgischen Eingriffs erzeugt wird, wobei das Verfahren Folgendes umfasst:
Bestimmen (444) der Position des zweiten Markierers (454, 456, 468) in dem Bezugsrahmen (452) aus der Position des zweiten Markierers, der von dem Verfolgungssystem verfolgt wird, während ein erstes Bild des Körperteils von einem abbildenden System an einem ersten Ort aufgenommen worden ist,
Bestimmen (446) der Position eines ersten Bildes (460) des Körperteils in dem Bezugsrahmen (458) des Verfolgungssystems aus der Position eines ersten Markierers,
der an einem Teil des abbildenden Systems angebracht ist und von dem Verfolgungssystem verfolgt wird, während das erste Bild des Körperteils aufgenommen worden ist, und einer bekannten Positionsbeziehung zwischen dem ersten Markierer und einer Bildebene des abbildenden Systems,
Bestimmen (448) einer Abbildung (468), um das erste Bild (460) in eine Ausrichtung mit der Position des Körperteils in dem Bezugsrahmen zu bringen, wobei die detektierte Position des zweiten Markierers in dem Bezugsrahmen verwendet wird,
Bestimmen (298, 396) der Position des Körperteils in dem Bezugsrahmen des Verfolgungssystems oder eines zweiten Verfolgungssystems aus der Position des zweiten Markierers, während sich der Körperteil in einem Operationssaal befindet, wobei es sich um einen anderen Ort als den ersten Ort handelt, und der von entweder dem Verfolgungssystem, das zu dem Operationssaal bewegt worden ist, oder einem zweiten Verfolgungssystem in dem Operationssaal verfolgt wird, und
Anzeigen (300, 398) des ausgerichteten Bildes, indem die Abbildung dazu verwendet wird, das erste Bild mit der detektierten Position des Körperteils in dem Operations-saal auszurichten.

38. Verfahren gemäß Anspruch 37, bei dem das erste Bild einen weiteren Körperteil des Patienten, der einen dritten von dem Verfolgungssystem detektierbaren Markierer trägt, enthält, und bei dem das Verfahren ferner ein Bestimmen der Position des dritten Markierers (456) in dem Bezugsrahmen umfasst.

39. Verfahren gemäß Anspruch 37, bei dem das Bestimmen der Position des ersten Bildes ein Bestimmen der Position der Bildebene (326) des abbildenden Systems relativ zu einem Patientenstützteil (328) des abbildenden Systems aufweist.

40. Verfahren gemäß einem der Ansprüche 37, 38 oder 39, bei dem das Bestimmen der Position eines ersten Bildes des Körperteils in dem Bezugsrahmen ein Bestimmen der Position eines Bildes (466) von zumindest einem Teil des zweiten Markierers in dem ersten Bild (460) aufweist.

41. Computerlesbares Medium, das einen, von einer Datenverarbeitungsvorrichtung ausführbaren Computerprogrammcode trägt, um ein Verfahren gemäß einem der Ansprüche 38 bis 40 bereitzustellen.

## Revendications

1. Procédé (250, 350, 370) de génération et d'affichage d'une image enregistrée d'une partie du corps d'un patient destinée à être utilisée lors d'une procédure chirurgicale assistée par ordinateur, le procédé consistant à :
capturer (256, 356, 376) au moins une première image de la partie du corps, en utilisant un système d'imagerie (202, 322) au niveau d'un premier emplacement, dans lequel un premier repère (210, 336) détectable par un système de suivi (132) présentant une trame de référence est attaché à une partie du système d'imagerie ;
détecter (254, 354, 374) la position d'un deuxième repère (216, 218), lequel a été attaché à la partie du corps, avant des quelconques étapes chirurgicales de la procédure chirurgicale, tandis que ladite au moins une première image est capturée en utilisant le système de suivi ;
détecter (354, 354, 374) la position du premier repère (210, 336) tandis que ladite au moins une première image est capturée en utilisant le système de suivi ;
obtenir (446) une indication de la position de la première image dans la trame de référence du système de suivi, en utilisant la position détectée du premier repère et une relation de positionnement connue entre le premier repère et un plan d'image du système d'imagerie ;
déterminer (448) une mise en correspondance (461) en vue d'amener la première image (460) en alignement avec la position de la partie du corps dans la trame de référence, en utilisant la position détectée (444) du deuxième repère dans la trame de référence ;
détecter (298, 386) la position du deuxième repère tandis que la partie du corps se situe dans une salle d'opération, laquelle correspond à un emplacement distinct du premier emplacement, en utilisant soit le système de suivi qui a été déplacé vers la salle d'opération à partir du premier emplacement, soit un second système de suivi, et déterminer la position de la partie du corps dans la trame de référence du système de suivi ou du second système de suivi ; et
afficher (300, 398) l'image enregistrée en utilisant la mise en correspondance en vue d'aligner la première image avec la position détectée de la partie du corps dans la salle d'opération tandis que la partie du corps est dans la salle d'opération.

2. Procédé selon la revendication 1, dans lequel la première image (460) inclut le deuxième repère (466) et au moins une partie de la partie du corps, et dans lequel la position du deuxième repère dans la première image est utilisée en vue de déterminer la mise en correspondance (461).

3. Procédé selon la revendication 2, dans lequel la procédure chirurgicale (290) est une procédure chirurgicale orthopédique.

4. Procédé selon l'une quelconque des revendications précédentes, dans lequel le deuxième repère (216, 218) est détectable par voie hertzienne à des fréquences radio, par le système de suivi.

5. Procédé selon l'une quelconque des revendications précédentes, dans lequel le système d'imagerie (202) est un système à rayons X.

6. Procédé selon la revendication 5, dans lequel la position du deuxième repère est détectée alors que le patient (214) est debout.

7. Procédé selon l'une quelconque des revendications précédentes, dans lequel le deuxième repère (216, 218) est suivi par voie hertzienne au moyen d'un système de suivi magnétique (132).

8. Procédé selon l'une quelconque des revendications 9 à 11, dans lequel le premier repère (210) est attaché à un détecteur de rayons X (208) et le procédé consiste à déterminer la position du détecteur de rayons X (280) dans la trame de référence du système de suivi (132).

9. Procédé selon l'une quelconque des revendications 5 à 8, consistant en outre à capturer une seconde image de la partie du corps au moyen du système à rayons X (202), et dans lequel la seconde image se trouve dans une seconde direction distincte d'une première direction dans laquelle la première image a été capturée.

10. Procédé selon la revendication 9, dans lequel la capture de la seconde image consiste à déplacer le patient (214) relativement au système à rayons X (202).

11. Procédé selon la revendication 10, dans lequel la capture de la seconde image consiste à déplacer une source de rayons X (204) relativement au patient (214), et le procédé consiste en outre à utiliser le système de suivi (132) en vue de déterminer la position de la source de rayons X (204) dans la trame de référence du système de suivi lorsque la seconde image est capturée.

12. Procédé selon la revendication 9, dans lequel la première image est capturée en utilisant une première source de rayons X (204) et dans lequel la capture de la seconde image consiste à utiliser une seconde source de rayons X (204') au niveau d'une seconde position qui est distincte d'une première position de la première source de rayons X.

13. Procédé selon la revendication 9, consistant en outre à générer une image tridimensionnelle de la partie du corps à partir des première et seconde images.

14. Procédé selon la revendication 5, consistant en outre à déterminer la distance (d) entre la partie du corps (214) et un plan d'imagerie d'un détecteur de rayons X (208) le long d'une direction perpendiculaire au plan du plan d'imagerie, et à utiliser la distance en vue de corriger la première image capturée par le détecteur de rayons X.

15. Procédé selon l'une quelconque des revendications 1 à 4, dans lequel le système d'imagerie est un tomodensitogramme (322) ou un système d'imagerie par résonance magnétique.

16. Procédé selon la revendication 15, dans lequel la partie du corps du patient (214) est située sur une partie de prise en charge de patient (328) du système d'imagerie lorsque la première image est capturée, et consistant en outre à déterminer la position de la partie de prise en charge de patient (328) dans la trame de référence du système de suivi, en suivant le premier repère (336) au moyen du système de suivi.

17. Procédé selon la revendication 16, dans lequel le premier repère (336) est monté sur la partie de prise en charge de patient (328).

18. Procédé selon la revendication 15, dans lequel la partie du corps du patient est située sur une partie de prise en charge de patient du système d'imagerie lorsque la première image est capturée, et consistant en outre à déterminer la position du plan d'imagerie (326) du système d'imagerie relativement à la position de la partie de prise en charge de patient (328), à partir de la position suivie du premier repère (336).

19. Procédé selon la revendication 15, dans lequel la première image inclut le deuxième repère et au moins une partie de la partie du corps.

20. Procédé selon l'une quelconque des revendications précédentes, et dans lequel :
un troisième repère (218) détectable par le système de suivi a été attaché à une partie du corps supplémentaire avant des quelconques étapes chirurgicales de la procédure chirurgicale guidée par l'image ; et le procédé consiste en outre à détecter la position du troisième repère (218) dans la trame de référence.

21. Procédé selon la revendication 20, dans lequel la mise en correspondance de la première image en alignement avec la position de la partie du corps consiste à utiliser la position du troisième repère dans la trame de référence.

22. Système (130, 320) destiné à générer et à afficher une image enregistrée d'une partie du corps d'un patient (214) destinée à être utilisée lors d'une procédure chirurgicale guidée par l'image, le système incluant :
un système de suivi (132) destiné à détecter des repères et à déterminer la position des repères dans une trame de référence du système de suivi ;
un système d'imagerie (202, 322), situé à un premier emplacement, destiné à capturer au moins une première image de la partie du corps, dans lequel un premier repère (210, 336) détectable par le système de suivi est attaché à une partie du système d'imagerie ;
un deuxième repère (216, 218) pouvant être attaché à la partie du corps du patient (218) et détectable par le système de suivi ; et
un système de commande d'ordinateur (122) configuré de manière à :
amener le système d'imagerie à capturer (256, 356, 376) au moins une première image de la partie du corps ;
amener le système de suivi à détecter (254, 354, 374) la position du deuxième repère (216, 218) lorsque ce dernier est attaché à la partie du corps tandis que ladite au moins une première image est capturée au niveau du premier emplacement ;
amener le système de suivi à détecter (254, 354, 374) la position du premier repère (210, 336) tandis que ladite au moins une première image est capturée ;
obtenir une indication de la position de la première image dans la trame de référence du système de suivi, en utilisant la position détectée du premier repère et une relation de positionnement connue entre le premier repère (210, 336) et un plan d'image (326) du système d'imagerie ;
déterminer (448) comment mettre en correspondance la première image en alignement avec la position de la partie du corps dans la trame de référence, en utilisant la position détectée (444) du deuxième repère dans la trame de référence ;
amener, soit le système de suivi, lorsqu'il a été déplacé vers une salle d'opération, soit un système de suivi supplémentaire situé dans la salle d'opération, à détecter (298, 386) la position du deuxième repère tandis que la partie du corps se situe dans la salle d'opération, laquelle correspond à un emplacement distinct du premier emplacement, et déterminer la position de la partie du corps dans la trame de référence du système de suivi ou du système de suivi supplémentaire ; et
afficher (300, 398) l'image enregistrée en utilisant la mise en correspondance en vue d'aligner la première image avec la position détectée de la partie du corps dans la salle d'opération.

23. Système selon la revendication 22, dans lequel le deuxième repère (216, 218) est détectable par voie hertzienne par le système de suivi (132).

24. Système selon la revendication 23, dans lequel le deuxième repère (216, 218) est détectable par voie hertzienne, par le système de suivi (132), à des fréquences radio.

25. Système selon l'une quelconque des revendications 23 à 25, dans lequel le deuxième repère (216, 218) est implantable par voie percutanée dans un os du patient.

26. Système selon l'une quelconque des revendications 23 à 26, dans lequel le système d'imagerie (202) est un système d'imagerie à rayons X comprenant une source de rayons X (204) et un détecteur de rayons X (208) comportant le plan d'imagerie.

27. Système selon la revendication 26, dans lequel le premier repère (210) est monté sur le détecteur de rayons X (208).

28. Système selon la revendication 26 ou 27, comprenant en outre un troisième repère (206) détectable par le système de suivi, et dans lequel le troisième repère est monté sur la source de rayons X (204), et dans lequel la source de rayons X (204) est mobile relativement au détecteur de rayons X (208).

29. Système selon la revendication 26 ou 27, comprenant en outre :
un quatrième repère (206') détectable par le système de suivi ; et
une source de rayons X supplémentaire (204'), et dans lequel le quatrième repère est monté sur la source de rayons X supplémentaire.

30. Système selon l'une quelconque des revendications 26 à 29, dans lequel chaque repère est détectable magnétiquement et constitue un repère pouvant être suivi.

31. Système selon l'une quelconque des revendications 26 à 30, dans lequel le système de commande d'ordinateur (122) est en outre configuré de manière à déterminer une séparation (d) entre le deuxième repère (216, 218) et un plan d'imagerie du détecteur de rayons X (208), et à utiliser la séparation en vue de corriger l'agrandissement de la première image.

32. Système selon l'une quelconque des revendications 22 à 27, dans lequel le système d'imagerie est un tomodensitogramme ou un système d'imagerie par résonance magnétique (322).

33. Système selon la revendication 32, dans lequel le système d'imagerie inclut une partie de prise en charge de patient (328) et le premier repère (336) est monté sur la partie de prise en charge de patient.

34. Système selon la revendication 33, dans lequel le système de commande d'ordinateur (122) est en outre configuré de manière à déterminer la position du patient (214) lorsque la première image inclut le deuxième repère et au moins une partie de la partie du corps.

35. Système selon la revendication 33, dans lequel le système d'imagerie inclut une partie de prise en charge de patient (328) et dans lequel le système de commande d'ordinateur (122) détermine la position d'un plan d'imagerie (326) du système d'imagerie relativement à la partie de prise en charge de patient (328).

36. Système selon l'une quelconque des revendications 26 à 35, comprenant en outre un troisième repère (218) pouvant être attaché à une partie supplémentaire du corps du patient (214) et détectable par le système de suivi (132).

37. Procédé mis en oeuvre par ordinateur (440) en vue de générer et d'afficher une image enregistrée d'une partie du corps d'un patient (214) portant un deuxième repère (216, 218) détectable par un système de suivi (132) présentant une trame de référence, l'image enregistrée étant destinée à être utilisée dans une procédure chirurgicale guidée par l'image et étant générée préalablement à de quelconques étapes chirurgicales de la procédure chirurgicale guidée par l'image, le procédé consistant à :
déterminer (444) la position du deuxième repère (454, 456, 468) dans la trame de référence (452), à partir de la position du deuxième repère suivi par le système de suivi, tandis qu'une première image de la partie du corps a été capturée par un système d'imagerie à un premier emplacement ;
déterminer (446) la position d'une première image (460) de la partie du corps dans la trame de référence (458) du système de suivi, à partir de la position d'un premier repère attaché à une partie du système d'imagerie et suivi par le système de suivi, tandis que la première image de la partie du corps a été capturée, et à partir d'une relation de positionnement connue entre le premier repère et un plan d'image du système d'imagerie ;
déterminer (448) une mise en correspondance (461) en vue d'amener la première image (460) en alignement avec la position de la partie du corps dans la trame de référence, en utilisant la position détectée du deuxième repère dans la trame de référence ;
déterminer (298, 396) la position de la partie du corps dans la trame de référence du système de suivi ou d'un second système de suivi à partir de la position du deuxième repère, tandis que la partie du corps est située dans une salle d'opération, correspondant à un emplacement distinct du premier emplacement, et est suivi soit par le système de suivi qui a été déplacé vers la salle d'opération soit par un second système de suivi situé dans la salle d'opération ; et
afficher (300, 398) l'image enregistrée en utilisant la mise en correspondance en vue d'aligner la première image avec le position détectée de la partie du corps dans la salle d'opération.

38. Procédé selon la revendication 37, dans lequel la première image inclut une partie supplémentaire du corps du patient portant un troisième repère détectable par le système de suivi, et le procédé consiste en outre à déterminer la position du troisième repère (456) dans la trame de référence.

39. Procédé selon la revendication 37, dans lequel la détermination de la position de la première image consiste à déterminer la position du plan d'imagerie (326) du système d'imagerie relativement à une partie de prise en charge de patient (328) du système d'imagerie.

40. Procédé selon la revendication 37, 38 ou 39, dans lequel la détermination de la position d'une première image de la partie du corps dans la trame de référence consiste à déterminer la position d'une image (466) d'au moins une partie du deuxième repère au sein de la première image (460).

41. Support lisible par ordinateur portant un code de programme informatique exécutable par un dispositif de traitement de données en vue de fournir un procédé selon l'une quelconque des revendications 38 à 40.
